# EUROPEAN PATENT APPLICATION

(11) **EP 4 227 684 A1**
(43) Date of publication of application: **16.08.2023**
(21) Application number: 22305149.1
(22) Date of filing: 10.02.2022
(51) Int. Cl.: G01N 33/569, G01N 33/68

(54) **METHOD FOR INDUCING NEUTROPHIL EXTRACELLULAR TRAP (NET) FORMATION**

(71) Applicant: Tridek-One Therapeutics, 75005 Paris (FR); INSERM (Institut National de la Santé et de la Recherche Médicale), 75013 Paris (FR); Université Paris XIII, 93430 Villetaneuse (FR)
(72) Inventor: CALIGIURI, Giuseppina, 75018 Paris (FR); NICOLETTI, Antonino, 75018 Paris (FR)
(74) Representative: Icosa

(57) **Abstract**

The present invention relates to *in vitro* assay for inducing neutrophil extracellular trap (NET) formation, comprising contacting neutrophils in *in vitro* culture with immobilized human polyvalent immunoglobulins; and to uses thereof, in particular in an *in vitro* method for screening a drug for its ability to modulate NET formation, in an *in vitro* method for assessing the susceptibility of a subject to NET formation, in an *in vitro* method for predicting the response of a subject to a modulator of NET formation, and in *in vitro* method for monitoring the response of a subject to an immunomodulator.

## Description

### FIELD OF INVENTION

The present invention relates to the field of immunology and, more particularly, to the field of extracellular traps released by leukocytes, in particular by neutrophils.

### BACKGROUND OF INVENTION

Neutrophils represent up to 40 to 70% of leukocytes in humans and they are the first cell type recruited to the inflammatory site. As part of the innate immune system, they act as the first line of defense against pathogens. The mechanisms used by neutrophils to eliminate invading microorganisms include phagocytosis, reactive oxygen species (ROS) release and degranulation of microbicidal molecules. In 2004, an additional antimicrobial activity was identified: neutrophils are able to extrude a meshwork constituted of chromatin fibers, DNA, histones and granule-derived antimicrobial peptides and enzymes. These structures, called neutrophil extracellular traps (NETs), represent an important strategy to immobilize and kill pathogens.

Two mechanisms of NETs release have been identified: i) extrusion of mitochondrial DNA and serine protease from intact neutrophils, as opposed to ii) release of decondensed chromatin and granular contents into the extracellular space after dissolution of the nuclear and granular membranes, decondensation of the nuclear contents into the cytoplasm and rupture of the plasma membrane. The second mechanism is called NETosis and represents a distinct form of active cell death. NETosis can be triggered by diverse signals such as microbe-associated, inflammatory or endogenous molecules.

Even though NETs are involved in pathogen clearance, excessive NET formation can promote inflammation and tissue damage. A deleterious role of NETs has thus been identified, for example in chronic inflammatory diseases, as well as in cancer. Consequently, elucidation of the mechanisms behind NET formation has become an increasingly important topic.

Assays have thus been developed to study NET release *in vitro.* The most frequently used compound in *in vitro* studies is phorbol 12-myristate 13-acetate (PMA), a synthetic activator of the ubiquitous signal transduction enzyme protein kinase C (PKC) which leads to the phosphorylation of gp91phox/Nox2 (NADPH oxidase 2). However, while PMA efficiently promotes ROS production and NET formation by neutrophils *in vitro,* ROS production and NET formation induced by PMA are not physiologically relevant, since they bypass membrane receptors and their downstream signaling intermediates. Both ROS production and NET formation induced by physiological stimuli depend on discrete signaling pathways involving Src family kinases and the spleen tyrosine kinase also known as SYK (van der Linden M et al. Sci Rep. 2017 Jul 26;7(1):6529), which can be activated by cell surface receptors, and in particular by the Fc receptor. Therefore, there is still a need to identify physiologically relevant inducers of NETs to be used in *in vitro* assays.

The Inventors surprisingly discovered that immobilized human polyvalent immunoglobulins (also known as intravenous immunoglobulins or IVIg) are potent physiological inducers of NET formation by neutrophils. Indeed, the data included hereinafter demonstrate that NET formation induced by immobilized human polyvalent immunoglobulins depends on SYK signaling. The present invention thus relates to an *in vitro* method for inducing NET formation by contacting neutrophils in *in vitro* culture with immobilized human polyvalent immunoglobulins, and to uses of said method.

### SUMMARY

The present invention relates to an *in vitro* method for inducing neutrophil extracellular trap (NET) formation, said method comprising contacting neutrophils in *in vitro* culture with human polyvalent immunoglobulins, wherein said human polyvalent immunoglobulins are immobilized on a solid support. In some embodiments, the method does not comprise contacting the neutrophils in *in vitro* culture with phorbol-12-myristate-13-acetate (PMA).

In some embodiments, the solid support is coated with human polyvalent immunoglobulins using a coating solution comprising human polyvalent immunoglobulins at a concentration of at least about 3 µg/mL. In some embodiments, the neutrophils are cultured at a concentration of at least about 1x10⁵ neutrophils/mL of culture medium.

In some embodiments, the neutrophils are cultured in a culture medium comprising a cell-impermeant nucleic acid dye. In some embodiments, the method comprises assessing NET formation by image analysis. In some embodiments, the cell-impermeant nucleic acid dye is fluorescent and the method comprises assessing NET formation by fluorescence detection.

The present invention also relates to an *in vitro* method for screening a drug for its ability to modulate neutrophil extracellular trap (NET) formation, said method comprising:
a) contacting neutrophils in *in vitro* culture with a drug;
b) inducing NET formation by contacting the neutrophils in *in vitro* culture with human polyvalent immunoglobulins immobilized on a solid support according to the method described above; and
c) assessing the effect of the drug on NET formation in the *in vitro* culture of neutrophils,
wherein the order of step a) and b) can be inverted.

The present invention also relates to *in vitro* method for assessing the susceptibility of a subject to neutrophil extracellular trap (NET) formation, said method comprising:
a) contacting an *in vitro* culture of neutrophils previously obtained from a subject with human polyvalent immunoglobulins immobilized on a solid support, thereby inducing NET formation according to the method as described above; and
b) quantifying NET formation induced in the *in vitro* culture of neutrophils, thereby assessing the susceptibility of the subject to NET formation.
In some embodiments, the method for assessing the susceptibility of a subject to NET formation further comprises determining the ratio of neutrophils:human polyvalent immunoglobulins required to induce maximal NET formation in the *in vitro* culture of neutrophils.

The present invention also relates to *in vitro* method for predicting the response of a subject to a modulator of neutrophil extracellular trap (NET) formation, said method comprising:
a) contacting an *in vitro* culture of neutrophils previously obtained from a subject with a modulator of NET formation;
b) inducing NET formation by contacting the *in vitro* culture of neutrophils with human polyvalent immunoglobulins immobilized on a solid support according to the method as described above; and
c) assessing the effect of the modulator of NET formation on NET formation in the *in vitro* culture of neutrophils,
wherein the order of step a) and b) can be inverted.

The present invention further relates to *in vitro* method for monitoring the response of a subject to an immunomodulator, said method comprising:
a) contacting an *in vitro* culture of neutrophils previously obtained from a subject with human polyvalent immunoglobulins immobilized on a solid support, thereby inducing NET formation according to the method as described above, wherein said subject was treated or is being treated with an immunomodulator;
b) quantifying NET formation induced in the *in vitro* culture of neutrophils; and
c) comparing the NET formation induced in the *in vitro* culture of neutrophils to a reference value, thereby monitoring the response of the subject to the immunomodulator.
In some embodiments, the reference value is the NET formation induced in an *in vitro* culture of neutrophils previously obtained from the subject before treatment with the immunomodulator.

In some embodiments, the subject is suffering from a disease or condition to which NET formation may contribute, in particular selected from an inflammatory disease, a thromboembolic diseases, a solid cancer, and a fibrosis. In some embodiments, the disease or condition to which NET formation may contribute is selected from vasculitis, in particular ANCA vasculitis; asthma; rheumatoid arthritis (RA); chronic obstructive pulmonary disease (COPD); systemic lupus erythematous (SLE); systemic sclerosis; adverse cardiovascular events such as myocardial infarction; ischemic stroke; atherosclerosis; venous thrombosis; cancer, in particular solid cancer; fibrosis, for example cystic fibrosis; sepsis; gout; and Alzheimer's disease.

### DEFINITIONS

In the present invention, the following terms have the following meanings:
"**Ig**" refers to immunoglobulin(s).
"**About**" preceding a figure encompasses plus or minus 10%, or less, of the value of said figure. It is to be understood that the value to which the term "about" refers is itself also specifically, and preferably, disclosed.
"**Biological sample**" refers to a cell, tissue or organ. Examples of biological samples include bodily fluids (preferably blood), whole blood, apheresis blood, serum, plasma, urine, feces, synovial fluid, bronchoalveolar lavage fluid, sputum, lymph, ascitic fluids, urine, amniotic fluid, peritoneal fluid, cerebrospinal fluid, pleural fluid, pericardial fluid, and alveolar macrophages, tissue lysates, biopsies and extracts prepared from diseased tissues.
"**DNA**" refers to deoxyribonucleic acid.
"**Fc**" or "**fragment crystallizable**" refers to the tail region of an immunoglobulin that interacts with cell surface receptors called Fc receptors.
"**Measuring**" or "**measurement**", or alternatively "**detecting**" or "**detection**", mean assessing the presence, absence, level, or quantity of a given object, *e.g*., NET formation. "Measuring" or "measurement", or alternatively "detecting" or "detection" as used herein include the derivation of the qualitative or quantitative assessment of NET formation.
"**Subject**" refers to an animal, preferably a mammal, more preferably a human. In some embodiments, a subject may be a "patient", who/which is awaiting the receipt of, or is receiving medical care or was/is/will be the object of a medical procedure, or is monitored for the development of the targeted disease or condition, such as, for example, a neutrophil-associated disease or condition, in particular a disease or condition to which NET formation may contribute. In some embodiments, the subject is an adult (for example a subject above the age of 18). In some embodiments, the subject is a child (for example a subject below the age of 18). In some embodiments, the subject is a male. In some embodiments, the subject is a female. In some embodiments, the subject is affected, preferably is diagnosed, with a neutrophil-associated disease or condition, in particular with a disease or condition to which NET formation may contribute. In some embodiments, the subject is at risk of developing a neutrophil-associated disease or condition, in particular with a disease or condition to which NET formation may contribute. Examples of risks factor include genetic predisposition, susceptibility to NET formation, or familial history of neutrophil-associated diseases.
"**Treating**" or "**treatment**" or "**alleviation**" refers to a therapeutic treatment, to a prophylactic (or preventative) treatment, or to both a therapeutic treatment and a prophylactic (or preventative) treatment; wherein the object is to prevent or slow down (lessen) the targeted pathologic condition or disorder. Those in need of treatment include those already with the disorder as well as those prone to have the disorder or those in whom the disorder is to be prevented. In some embodiments, a subject is successfully "treated" for a disease or disorder if, after receiving a therapeutic amount of a therapeutic agent, such as a NETosis modulator or an immunomodulator, the subject shows at least one of the following: relief to some extent of one or more of the symptoms associated with the disease or disorder to be treated, and/or improvement in quality-of-life issues. The above parameters for assessing successful treatment and improvement in the disease or disorder are readily measurable by routine procedures familiar to a physician.

### DETAILED DESCRIPTION

The present invention firstly relates to an *in vitro* method for inducing extracellular trap (ET) formation, comprising contacting cells in *in vitro* culture with human polyvalent immunoglobulins immobilized on a solid support.

"**Extracellular Traps**" or "**ETs**" refer to lacy structures constituted of a DNA backbone embedded with antimicrobial peptides, proteases and histones. Extracellular traps can be released by innate immune cells and adaptive immune cells, including neutrophils, eosinophils, basophils, macrophages, mast cells, plasmacytoid dendritic cells, B lymphocytes and T lymphocytes. Extracellular trap release allows to immobilize and kill invading microorganisms in the extracellular milieu. Extracellular trap formation can be of two types: with or without the death of the producer cell. The cell death program associated with extracellular traps formation is called ETosis.

Another object of the present invention is an *in vitro* method for inducing ETosis, comprising contacting cells in *in vitro* culture with human polyvalent immunoglobulins immobilized on a solid support.

In some embodiments, the *in vitro* method of the present invention for inducing extracellular trap (ET) formation, comprising contacting cells in *in vitro* culture with human polyvalent immunoglobulins immobilized on a solid support, also induces NETosis.

As indicated above, ET release and/or ETosis can be induced in a number of immune cells, in particular in immune cells expressing Fc receptors at their surface, such as neutrophils, eosinophils, basophils, macrophages, mast cells, dendritic cells (in particular plasmacytoid dendritic cells), B lymphocytes (or B cells) and T lymphocytes (or T cells).

"**Granulocytes**" refers to specific leukocytes characterized by the presence of cytoplasmic granules containing lytic enzymes and a poly-lobed nucleus. Granulocytes represent the most abundant cell type in human peripheral blood, belong to the myeloid cell family, and are part of the innate immune system. Depending on the content of their cytoplasmic granules, granulocytes are classified as neutrophils, eosinophils or basophils. Granulocytes are important in the removal of bacteria and parasites from the body.

"**Neutrophils**", "**Polymorphonuclear neutrophils (PMNs)**" or **"Neutrophilic granulocytes**" refer to the most abundant type of granulocytes in human. Neutrophils are the first type of leukocytes recruited at the injury or infection sites, in response to CXCL8 produced by stressed epithelial cells and tissue-resident macrophages. Neutrophils represent the first line of defense against invading pathogens, by phagocytosis of microorganisms, release of antimicrobial factors contained in cytoplasmic granules, production of reactive oxygen species or extrusion of neutrophil extracellular traps (NETs). Neutrophils' granules contain bactericidal enzymes such as lysozyme, myeloperoxidase, neutral proteases, acid hydrolases, collagenase, lactoferrin and cathepsin B, which possess potent antimicrobial activity but are also highly cytotoxic.

"**Eosinophils**", "**Polymorphonuclear eosinophils**" or "**Eosinophilic granulocytes**" refer to a variety of granulocytes implicated in host defense against nematodes and other parasitic infections thanks to their capacities of phagocytosis, degranulation and reactive oxygen species (ROS) production. Additionally, eosinophils have been shown to release eosinophil extracellular traps (EETs). However, eosinophils' degranulation capacity can also be detrimental and participate in the inflammatory process of allergic diseases.

"**Basophils**", "**Polymorphonuclear basophils**" or "**Basophilic granulocytes**" refer to the least abundant type of granulocytes, which possesses a beneficial role in infections with helminth parasites. Basophils have important roles in the regulation of adaptive immune responses through their secretion of various cytokines, but also their capacity of acting as antigen-presenting cells. Basophils have also been shown to release basophil extracellular traps (BETs).

"**Macrophages**" are tissue-resident leukocytes involved in innate immune defense. They possess phagocytosis abilities, which allows them to eliminate pathogens and remove cell debris, and are involved in T lymphocytes activation initiation. An additional effector function has recently been described for macrophages: the ability to release macrophage extracellular traps (METs) composed of DNA, histones, myeloperoxidase and lysozymes.

"**Mast cells**" are tissue-resident leukocytes characterized by a monolobulated nucleus and granules containing histamine, heparin, tryptase and chymase. Mast cells are involved in immune responses to various pathogens and have been shown to have a role in allergy. Mast cells are also able to release mast cell extracellular traps (MCETs) comprising DNA, tryptase, histones and cathelicidins.

"**Dendritic cells**" or "**DC**" account for less than 1% of leukocytes in peripheral blood. Dendritic cells play a role in linking the innate and adaptive immune systems. They are sentinels in peripheral tissues, patrolling the presence of antigens for presentation to T lymphocytes. Dendritic cells, and in particular plasmacytoid dendritic cells (pDCs), have been recently shown to release dendritic cell extracellular traps (DCETs).

"**B lymphocytes**" or "**B cells**" represent about 15% of peripheral blood leukocytes and are involved in humoral immunity through their production of antibodies. B lymphocytes also have the ability to present antigens and produce cytokines. More recently, B lymphocytes have been shown to produce lymphocyte extracellular traps (LETs).

"**T lymphocytes**" or "**T cells**" are a specific type of leukocytes possessing important functions in directly killing infected host cells (*i.e*., cytotoxic T lymphocytes), activating other immune cells, producing cytokines and regulating the immune response (*i.e.,* helper T lymphocytes). Both cytotoxic and helper T lymphocytes have been shown to produce lymphocyte extracellular traps (LETs).

The cells to be contacted with human polyvalent immunoglobulins immobilized on a solid support according to the method described herein may be any cell expressing Fc receptors at its surface. In particular, said cells may be neutrophils, eosinophils, basophils, macrophages, mast cells, dendritic cells, B lymphocytes and/or T lymphocytes. In some embodiments, said cells are neutrophils and/or eosinophils. In some embodiments, said cells are neutrophils.

In some embodiments, the cells, preferably neutrophils, are human cells.

In some embodiments, the cells, preferably neutrophils, are isolated from a biological sample obtained from a subject. In some embodiments, the cells, preferably neutrophils, are isolated from a blood sample. As used herein, "blood" includes whole blood, plasma, serum, constituents, or any other derivative of blood. In some embodiments, the cells, preferably neutrophils, are isolated from a whole blood sample. In some embodiments, the cells, preferably neutrophils, are isolated from a peripheral blood sample.

In some embodiments, the cells, preferably neutrophils, are isolated from a biological sample as described above previously taken from a subject, *i.e.,* the methods of the present invention do not comprise an active step of recovering a sample from a subject. Consequently, according to some embodiments, the methods described herein are non-invasive methods, *i.e.,* the methods described herein are *in vitro* methods.

Methods used for isolating cells from a biological sample, in particular a blood sample, are well-known in the art. Methods used for isolating neutrophils from a biological sample, in particular a blood sample, are well-known in the art. For example, a standard density gradient separation method to isolate human neutrophils from whole blood is described in Oh et al., J Vis Exp. 2008, 23;(17):745. Additionally, commercial kits are available, such as: EasySep^{™} Direct Human Neutrophil Isolation Kit (Catalog # 19666, Stem Cell Technologies), MACSxpress^{®} Whole Blood Neutrophil Isolation Kit, human (Catalog # 130-104-434, Miltenyi Biotec).

The present invention further relates to an *in vitro* method for inducing neutrophil extracellular trap (NET) formation, comprising contacting neutrophils in *in vitro* culture with human polyvalent immunoglobulins immobilized on a solid support.

"**Neutrophil Extracellular Traps**" or "**NETs**" refer to complexes comprising large strands of decondensed DNA, histones and granular proteins which are released by neutrophils. The primary function of NETs is the trapping of extracellular microorganisms. NETs capture bacteria and fungi while simultaneously providing high local concentrations of anti-microbial components, thus killing pathogens extracellularly. NET formation can be triggered by various molecular mechanisms including receptor-mediated signals (such as TLR engagement), or calcium channel formation through ionophores. NETs are also highly toxic to the host and NET release contributes to cellular injury and organ dysfunction.

Another object of the present invention is an *in vitro* method for inducing NETosis, comprising contacting neutrophils in *in vitro* culture with human polyvalent immunoglobulins immobilized on a solid support.

In some embodiments, the *in vitro* method of the present invention for inducing NET formation, comprising contacting neutrophils in *in vitro* culture with human polyvalent immunoglobulins immobilized on a solid support, also induces NETosis.

"**NETosis**" refers to the specific cell death program which neutrophils may undergo following NET formation. After receiving a signal inducing NET formation, a cascade of events is initiated, leading to chromatin decondensation, nuclear membrane disintegration, mixing of decondensed DNA with granules and cytosolic proteins and perforation of the plasma membrane. Finally, meshes composed of nuclear material, associated with cytoplasmic and nuclear proteins are extruded by neutrophils in the extracellular space.

The cells, preferably neutrophils, to be contacted with human polyvalent immunoglobulins immobilized on a solid support according to the method described herein may be cultured in any suitable culture medium. Suitable culture media are well-known to one skilled in the art and may be selected, for example, depending on the cells. Examples of suitable media include HBSS (Hank's Buffered Saline Solution) medium and RPMI (Roswell Park Memorial Institute) 1640 medium. The culture medium may be supplemented with additional substances such as serum and serum components, vitamins, reducing agents, and/or buffering agents.

In some embodiments, the cells, preferably neutrophils, are cultured in HBSS medium containing calcium and magnesium (said medium is commercially available, for example from Gibco (reference 24020-091)).

The cells, preferably neutrophils, may be cultured under any suitable culture conditions, for example with regards to temperature, humidity, CO₂. Suitable culture conditions are well-known to one skilled in the art and may be selected, for example, depending on the cells. In some embodiments, the cells, preferably neutrophils, are cultured in culture conditions suitable for human cells. In some embodiments, the cells, preferably neutrophils, are cultured at 37°C and 5% CO₂.

In some embodiments, the cells, preferably neutrophils, are cultured for at least 3 hours. In some embodiments, the cells, preferably neutrophils, are cultured for at least 4, 5, 6, 7, 8, 9, 10, 12, 15, 18, or 24 hours.

Advantageously, the methods described herein do not involve contacting cells, preferably neutrophils, with phorbol-12-myristate-13-acetate (PMA). Therefore, in some embodiments, the methods described herein do not comprise contacting the cells, preferably neutrophils, in *in vitro* culture with PMA.

In some embodiments, the cells, preferably neutrophils, are cultured at a concentration of at least about 1x10⁴, 5×10⁴, 1x10⁵, 5×10⁵, 1×10⁶, 5×10⁶, 1×10⁷, or 5×10⁷ cells per milliliter of culture medium, preferably at a concentration of at least about 1x10⁶ cells/mL of culture medium. In some embodiments, the cells, preferably neutrophils, are cultured at a concentration ranging from about 1x10⁴ to about 5×10⁷ cells/mL of culture medium, preferably from about 1x10⁵ to about 1x10⁷ cells/mL of culture medium, more preferably from about 5×10⁵ to about 5×10⁶ cells/mL of culture medium. In some embodiments, the cells, preferably neutrophils, are cultured at a concentration of about 1x10⁴, 5×10⁴, 1x10⁵, 5×10⁵, 1x10⁶, 5×10⁶, 1x10⁷, or 5×10⁷ cells/mL of culture medium, preferably at a concentration of 1×10⁶ cells/mL of culture medium.

In some embodiments, the cells, preferably neutrophils, are cultured at a density of at least about 5×10³, 7.5×10³, 1x10⁴, 2.5×10⁴, 5×10⁴, 7.5×10⁴, 1x10⁵, 2.5×10⁵, 5×10⁵, 7.5×10⁵, or 1x10⁶ cells per well in a 96-well culture plate, preferably at a density of at least about 7.5×10⁴ cells per well in a 96-well culture plate. In some embodiments, the cells, preferably neutrophils, are cultured at a density ranging from about 5×10³ to about 5×10⁵ cells per well in a 96-well culture plate, preferably from about 7.5×10³ to about 2.5x10⁵ cells per well in a 96-well culture plate, more preferably from about 1×10⁴ to about 1x10⁵ per well in a 96-well culture plate. In some embodiments, the cells, preferably neutrophils, are cultured at a density of about 5×10³, 7.5×10³, 1x10⁴, 2.5×10⁴, 5×10⁴, 7.5×10⁴, 1x10⁵, 2.5x10⁵, 5×10⁵, 7.5×10⁵, or 1x10⁶ cells per well in a 96-well culture plate, preferably at a density of about 7.5×10⁴ cells per well in a 96-well culture plate.

In some embodiments, the cells, preferably neutrophils, are cultured at a concentration or density equivalent to a density of at least about 5×10³, 7.5×10³, 1×10⁴, 2.5×10⁴, 5×10⁴, 7.5×10⁴, 1x10⁵, 2.5×10⁵, 5×10⁵, 7.5×10⁵, or 1x10⁶ cells per well in a 96-well culture plate, preferably at a concentration or density equivalent to a density of at least about 7.5×10⁴ cells per well in a 96-well culture plate. In some embodiments, the cells, preferably neutrophils, are cultured at a concentration or density equivalent to a density ranging from about 5×10³ to about 5×10⁵ cells per well in a 96-well culture plate, preferably from about 7.5×10³ to about 2.5×10⁵ cells per well in a 96-well culture plate, more preferably from about 1x10⁴ to about 1x10⁵ per well in a 96-well culture plate. In some embodiments, the cells, preferably neutrophils, are cultured at a concentration or density equivalent to a density of about 5×10³, 7.5×10³, 1x10⁴, 2.5×10⁴, 5×10⁴, 7.5×10⁴, 1x10⁵, 2.5×10⁵, 5×10⁵, 7.5×10⁵, or 1x10⁶ cells per well in a 96-well culture plate, preferably at a concentration or density equivalent to a density of about 7.5×10⁴ cells per well in a 96-well culture plate. In some embodiments, a density of about 7.5x10⁴ cells per well in a 96-well culture plate is equivalent to a concentration of about 1x10⁶ cells per milliliter of culture medium.

"**Polyvalent immunoglobulins**" or "**intravenous immunoglobulins**" or "**IVIg**" refers to preparations of pooled normal polyspecific immunoglobulins, mostly IgG, obtained from large numbers of donors. IVIg thus correspond to a mix of immunoglobulins extracted and usually prepared from the plasma of between 1,000 and 15,000 donors. IVIg preparations consist of polyclonal natural immunoglobulins secreted by plasma B cells in response to immune stimuli. IVIg may comprise immunoglobulins secreted in response to microbial antigens, self-antigens, and/or anti-idiotypic antibodies which recognize other immunoglobulins. Clinically IVIg are widely used in replacement therapy in primary immunodeficiency syndromes and in secondary immunodeficiencies, as well as for the prevention and treatment of infectious diseases. Furthermore, IVIg are also used for immune modulation of patients with autoimmune and immune-complex diseases. IVIg are commercially available from several sources, for example from CSL Behring (King of Prussia, Pennsylvania, USA), Bio Products Laboratory (Elstree, United Kingdom), Octapharma (Lachen, Switzerland), LFB (les Ulis, France), Kedrion Biopharma Inc. (Barga, Italy), or Takeda Pharmaceuticals (Tokyo, Japan). In some embodiments, the IVIg are prepared from human donors. Therefore, in some embodiments, the polyvalent immunoglobulins are human polyvalent immunoglobulins.

In some embodiments, the IVIg are a 100 mg/mL solution for infusion, for example obtained from Privigen (CSL Behring UK Limited).

The solid support on which the IVIg are immobilized may be any vessel suitable for cell culture. Thus, in some embodiments, the solid support is a cell culture vessel (or culture vessel), in particular a sterile cell culture vessel. Examples of cell culture vessels include cell culture dishes, cell culture plates, cell culture flasks, cell culture bottles, cell culture tubes. In some embodiments, the solid support is a cell culture plate (or culture plate). In some embodiments, the solid support is a cell culture dish (or culture dish).

In some embodiments, the solid support is a multiple-well cell culture plate. Examples of multiple-well cell culture plates include 4-well, 6-well, 8-well, 12-well, 24-well, 48-well, and 96-well cell culture plates. In some embodiments, the solid support is a 4-well, 6-well, 8-well, 12-well, 24-well, 48-well, or a 96-well cell culture plate.

In some embodiments, the solid support is a culture vessel, for example a culture plate, suitable for fluorescence microscopy. In some embodiments, the solid support is a culture vessel, for example a culture plate, suitable for live fluorescence microscopy.

In some embodiments, the solid support has been exposed to a plasma gas in order to increase its hydrophobicity. In some embodiments, the solid support has increased protein binding capacity. In some embodiments, the solid support is high-binding. In some embodiments, high-binding corresponds to a binding capacity of about 400 to 500 ng IgG/cm².

In some embodiments, the solid support is a high-binding, sterile 96-well plate suitable for fluorescence microscopy.

The solid support on which the IVIg are immobilized may also be any beads suitable for cell culture, in particular for culture of neutrophils. Thus, in some embodiments, the solid support is beads, preferably phagocytosis-resistant beads. By phagocytosis-resistant, it is meant that the beads cannot be taken in by the cells, in particular neutrophils, via phagocytosis. In some embodiments, the beads have a diameter greater than about 30 µm. In some embodiments, the beads are polystyrene beads. In some embodiments, the beads are Epoxy beads.

In some embodiments, the solid support as described above is coated with human polyvalent immunoglobulins. Thus, in some embodiments, the solid support as described above is or was previously treated with a coating solution comprising human polyvalent immunoglobulins in order to obtain a solid support coated with human polyvalent immunoglobulins.

Accordingly, in some embodiments, the method as described above further comprises a first step of treating a solid support with a coating solution comprising human polyvalent immunoglobulins in order to obtain a solid support coated with human polyvalent immunoglobulins, that it to say a solid support on which human polyvalent immunoglobulins are immobilized.

Thus, in some embodiments, the *in vitro* method for inducing ET formation, preferably NET formation, and/or for inducing ETosis, preferably NETosis, comprises:
- treating a solid support as described above with a coating solution comprising human polyvalent immunoglobulins; and
- contacting cells, preferably neutrophils, in *in vitro* culture with the solid support coated with human polyvalent immunoglobulins.

In some embodiments, the solid support is treated with a coating solution comprising human polyvalent immunoglobulins for about 1, 2, 3, 4, or 5 hours, preferably for about 2 hours. In some embodiments, the solid support is treated with a coating solution comprising with human polyvalent immunoglobulins at room temperature.

The coating solution may be any suitable buffer solutions, in particular buffer solutions suitable for cell culture. Examples of buffer solutions include PBS (phosphate buffer saline). In some embodiments, the coating solution is PBS. In some embodiments, the coating solution is DPBS (Dulbecco's Phosphate - Buffered Saline) containing no calcium and no magnesium (KC1 2.67 mM, KH₂PO₄ 1.47 mM, NaCl 137.9 mM, Na₂HPO₄-7H₂O 8.06 mM).

In some embodiments, the solid support is coated with human polyvalent immunoglobulins using a coating solution comprising human polyvalent immunoglobulins at a concentration of at least about 1 µg/mL, 3 µg/mL, 10 µg/mL, 20 µg/mL, 30 µg/mL, 40 µg/mL, 50 µg/mL, 60 µg/mL, 70 µg/mL, 80 µg/mL, 90 µg/mL, 100 µg/mL, 150 µg/mL, 200 µg/mL, 250 µg/mL, 300 µg/mL, 350 µg/mL, 400 µg/mL, 500 µg/mL, 600 µg/mL, 700 µg/mL, 800 µg/mL, 900 µg/mL, or 1000 µg/mL, preferably at a concentration of at least about 3 µg/mL. Thus, in some embodiments, the coating solution as described above comprises human polyvalent immunoglobulins at a concentration of at least about 1, 3, 10, 20, 30, 40, 50, 60, 70, 80, 90, 100, 150, 200, 250, 300, 350, 400, 500, 600, 700, 800, 900, or 1000 µg/mL, preferably at a concentration of at least about 3 µg/mL.

In some embodiments, the solid support is coated with human polyvalent immunoglobulins using a coating solution comprising human polyvalent immunoglobulins at a concentration ranging from about 1 µg/mL to about 1000 µg/mL, preferably from about 3 µg/mL to about 300 µg/mL, more preferably from about 10 µg/mL to about 100 µg/mL. Thus, in some embodiments, the coating solution as described above comprises human polyvalent immunoglobulins at a concentration ranging from about 1 µg/mL to about 1000 µg/mL, preferably from about 3 µg/mL to about 300 µg/mL, more preferably from about 10 µg/mL to about 100 µg/mL.

In some embodiments, the solid support is coated with human polyvalent immunoglobulins using a coating solution comprising human polyvalent immunoglobulins at a concentration of about 1 µg/mL, 3 µg/mL, 10 µg/mL, 20 µg/mL, 30 µg/mL, 40 µg/mL, 50 µg/mL, 60 µg/mL, 70 µg/mL, 80 µg/mL, 90 µg/mL, 100 µg/mL, 150 µg/mL, 200 µg/mL, 250 µg/mL, 300 µg/mL, 350 µg/mL, 400 µg/mL, 500 µg/mL, 600 µg/mL, 700 µg/mL, 800 µg/mL, 900 µg/mL, or 1000 µg/mL. Thus, in some embodiments, the coating solution as described above comprises human polyvalent immunoglobulins at a concentration of about 1, 3, 10, 20, 30, 40, 50, 60, 70, 80, 90, 100, 150, 200, 250, 300, 350, 400, 500, 600, 700, 800, 900, or 1000 µg/mL.

In some embodiments, ET formation and/or ETosis is detected using a cell-impermeant nucleic acid dye. In some embodiments, NET formation and/or NETosis is detected using a cell-impermeant nucleic acid dye.

Accordingly, in some embodiments, the cells, preferably neutrophils, are cultured in a culture medium comprising a cell-impermeant nucleic acid dye. In some embodiments, the cell-impermeant nucleic acid dye is fluorescent. Examples of fluorescent cell-impermeant nucleic acid dyes suitable for cell culture include Sytox Green.

In some embodiments, the method comprises assessing ET formation, preferably NET formation, and/or ETosis, preferably NETosis, by image analysis. In some embodiments, the method comprises assessing ET formation, preferably NET formation, and/or ETosis, preferably NETosis, by live image analysis. In some embodiments, the method comprises assessing ET formation, preferably NET formation, and/or ETosis, preferably NETosis, by continuous image acquisition. In some embodiments, the method comprises assessing ET formation, preferably NET formation, and/or ETosis, preferably NETosis, by image acquisition at specific time points.

In some embodiments, the method comprises assessing ET formation, preferably NET formation, and/or ETosis, preferably NETosis, by fluorescence detection. Methods for detecting fluorescence, in particular for quantitatively detecting fluorescence, are well-known to one skilled in the art. Examples of such methods include using a fluorometer, a plate reader or any other apparatus able to detect and quantify fluorescent light signals.

In some embodiments, the method comprises assessing ET formation, preferably NET formation, and/or ETosis, preferably NETosis, by fluorescent microscopy. In some embodiments, the method comprises assessing ET formation, preferably NET formation, and/or ETosis, preferably NETosis, by live cell fluorescent microscopy.

In some embodiments, the method comprises quantifying ET formation, preferably NET formation, and/or ETosis, preferably NETosis. In some embodiments, the method comprises quantifying ET formation, preferably NET formation, and/or ETosis, preferably NETosis, by computer-assisted image analysis.

In some embodiments, ET formation, preferably NET formation, can be detected after 1 hour, 2 hours, 3 hours, 4 hours, 5 hours, 6 hours, 7 hours, 8 hours, 9 hours, or 10 hours of culture of the cells, preferably neutrophils, in contact with human polyvalent immunoglobulins immobilized on a solid support.

Another object of the present invention is a kit comprising:
- a solid support as described above;
- a coating solution as described above;
- human polyvalent immunoglobulins as described above; and optionally
- instructions for use.
In some embodiments, implementing the kit allows to obtain a solid support coated with human polyvalent immunoglobulins, that is to say human polyvalent immunoglobulins immobilized on a solid support.

Another object of the present invention is a kit comprising:
- a solid support as described above;
- a coating solution comprising soluble human polyvalent immunoglobulins as described above; and optionally
- instructions for use.
In some embodiments, implementing the kit allows to obtain a solid support coated with human polyvalent immunoglobulins, that is to say human polyvalent immunoglobulins immobilized on a solid support.

By "**kit**" is intended any manufacture (e.g., a package or a container) comprising a solid support, a coating solution, human polyvalent immunoglobulins, and optionally instructions for use. The kit may be promoted, distributed, or sold as a unit for performing the methods described herein.

Another object of the present invention is an *in vitro* method for screening a drug for its ability to modulate neutrophil extracellular trap (NET) formation and/or NETosis comprising:
a) contacting neutrophils in *in vitro* culture with a drug;
b) inducing NET formation and/or NETosis by contacting the neutrophils in *in vitro* culture with human polyvalent immunoglobulins immobilized on a solid support as described above; and
c) assessing the effect of the drug on NET formation and/or NETosis in the *in vitro* culture of neutrophils,
wherein the order of step a) and b) can be inverted.

In some embodiments, the method comprises:
a) contacting neutrophils in *in vitro* culture with a drug;
b) inducing NET formation and/or NETosis by contacting the neutrophils in *in vitro* culture with human polyvalent immunoglobulins immobilized on a solid support as described above; and
c) assessing the effect of the drug on NET formation and/or NETosis in the *in vitro* culture of neutrophils.

In some embodiments, the method comprises:
a) Inducing NET formation and/or NETosis by contacting neutrophils in *in vitro* culture with human polyvalent immunoglobulins immobilized on a solid support as described above;
b) contacting the neutrophils in *in vitro* culture with a drug; and
c) assessing the effect of the drug on NET formation and/or NETosis in the *in vitro* culture of neutrophils.

In some embodiments, by "contacting neutrophils in *in vitro* culture with a drug" it is meant adding a drug to the culture medium in which the neutrophils are cultured.

In some embodiments, the method for screening a drug for its ability to modulate NET formation and/or NETosis further comprises comparing the NET formation and/or NETosis obtained in presence of the drug to a reference.

In some embodiments, the reference is the NET formation and/or NETosis obtained in the absence of the drug. In some embodiments, the reference is the NET formation and/or NETosis obtained in the presence of a drug known to inhibit or decrease NET formation and/or NETosis. Examples of drugs known to inhibit or decrease NET formation and/or NETosis include disulfiram; peptidyl arginine deiminase 4 (PAD4) inhibitors, such as Cl-amidine; neutrophil elastase (NE) inhibitors, such as sivelestat (also known as elaspol), alvelestat; DNases, such as DNase I; and anti-citrullinated protein antibodies (ACP) also known as therapeutic anti-citrullinated protein antibody (tACPA), such as CIT-013. In some embodiments, the reference is the NET formation and/or NETosis obtained in the presence of a drug known to stimulate or increase NET formation and/or NETosis. Examples of drugs known to stimulate or increase NET formation and/or NETosis include sulfasalazine (SSZ).

Another object of the present invention is an *in vitro* method for assessing the susceptibility of a subject to neutrophil extracellular trap (NET) formation and/or NETosis comprising:
a) contacting an *in vitro* culture of neutrophils previously obtained from a subject with human polyvalent immunoglobulins immobilized on a solid support, thereby inducing NET formation and/or NETosis;
b) quantifying NET formation and/or NETosis induced in the *in vitro* culture of neutrophils, thereby assessing the susceptibility of the subject to NET formation and/or NETosis.

In some embodiments, the method further comprises a first step of isolating neutrophils from a biological sample, such as a peripheral blood sample, previously obtained from a subject. As indicated above, methods for isolating neutrophils from a blood sample are commonly known. However, in some embodiments, the method does not comprise an active step of recovering a biological sample from the subject. Consequently, according to some embodiments, the method is a non-invasive method, *i.e.,* the method is an *in vitro* method.

In some embodiments, the method further comprises determining the minimal ratio of neutrophils:human polyvalent immunoglobulins required to induce NET formation and/or NETosis in the *in vitro* culture of neutrophils previously obtained from a subject. For example, the minimal ratio of neutrophils:human polyvalent immunoglobulins required to induce NET formation and/or NETosis may be determined by contacting *in vitro* cultures of a given concentration (*e.g*., about 1x10⁶ cells/mL of culture medium) of neutrophils previously obtained from a subject with increasing densities of human polyvalent immunoglobulins immobilized on a solid support.

In some embodiments, the method further comprises determining the ratio of neutrophils:human polyvalent immunoglobulins required to induce maximal NET formation and/or NETosis in the *in vitro* culture of neutrophils previously obtained from a subject. For example, the ratio of neutrophils:human polyvalent immunoglobulins required to induce maximal NET formation and/or NETosis may be determined by contacting *in vitro* cultures of a given concentration (*e.g*., about 1x10⁶ cells/mL of culture medium) of neutrophils previously obtained from a subject with increasing densities of human polyvalent immunoglobulins immobilized on a solid support.

In some embodiments, the susceptibility of a subject to NET formation and/or NETosis is determined based on the minimal ratio of neutrophils:human polyvalent immunoglobulins required to induce NET formation and/or NETosis in the *in vitro* culture of neutrophils previously obtained from a subject.

In some embodiments, the susceptibility of a subject to NET formation and/or NETosis is determined based on the ratio of neutrophils:human polyvalent immunoglobulins required to induce maximal NET formation and/or NETosis in the *in vitro* culture of neutrophils previously obtained from a subject.

For example, in some embodiments, it is considered that:
- when the ratio of neutrophils:human polyvalent immunoglobulins required to induce maximal NET formation and/or NETosis in the *in vitro* culture of neutrophils previously obtained from a subject is a high ratio of neutrophils:human polyvalent immunoglobulins, the subject is a high responder (that is to say the subject is very susceptible or shows high susceptibility to NET formation and/or NETosis);
- when the ratio of neutrophils:human polyvalent immunoglobulins required to induce maximal NET formation and/or NETosis in the *in vitro* culture of neutrophils previously obtained from a subject is an intermediate ratio of neutrophils:human polyvalent immunoglobulins, the subject is an intermediate responder (that is to say the subject is susceptible or shows susceptibility to NET formation and/or NETosis); and/or
- when the ratio of neutrophils:human polyvalent immunoglobulins required to induce maximal NET formation and/or NETosis in the *in vitro* culture of neutrophils previously obtained from a subject is a low ratio of neutrophils:human polyvalent immunoglobulins, the subject is a low responder (that is to say the subject is not very susceptible or shows little susceptibility to NET formation and/or NETosis).

In some embodiments, a high responder (that is to say a subject very susceptible or showing high susceptibility to NET formation and/or NETosis) corresponds to a subject showing maximal NET formation and/or NETosis at a low concentration or density of immobilized human polyvalent immunoglobulins and thus at a high ratio of neutrophils:human polyvalent immunoglobulins.

In some embodiments, a high ratio of neutrophils:human polyvalent immunoglobulins corresponds:
- to a culture of neutrophils at a concentration ranging from about 1x10⁵ to about 1x10⁷ cells/mL of culture medium, preferably from about 5×10⁵ to about 5×10⁶ cells/mL of culture medium, more preferably at a concentration of about 1×10⁶ cells/mL of culture medium; and
- to a solid support coated with a coating solution comprising human polyvalent immunoglobulins at a concentration ranging from about 1 µg/mL to about 20 µg/mL, preferably from about 3 µg/mL to about 15 µg/mL, more preferably at a concentration of about 10 µg/mL.

In some embodiments, an intermediate responder (that is to say a subject susceptible or showing susceptibility to NET formation and/or NETosis) corresponds to a subject showing maximal NET formation and/or NETosis at an intermediate concentration or density of immobilized human polyvalent immunoglobulins and thus at an intermediate ratio of neutrophils:human polyvalent immunoglobulins.

In some embodiments, an intermediate ratio of neutrophils:human polyvalent immunoglobulins corresponds for example:
- to a culture of neutrophils at a concentration ranging from about 1x10⁵ to about 1x10⁷ cells/mL of culture medium, preferably from about 5×10⁵ to about 5x10⁶ cells/mL of culture medium, more preferably at a concentration of about 1×10⁶ cells/mL of culture medium; and
- to a solid support coated with a coating solution comprising human polyvalent immunoglobulins at a concentration ranging from about 20 µg/mL to about 50 µg/mL, preferably from about 25 µg/mL to about 40 µg/mL, more preferably at a concentration of about 30 µg/mL.

In some embodiments, a low responder (that is to say a subject not very susceptible or showing little susceptibility to NET formation and/or NETosis) corresponds to a subject showing maximal NET formation and/or NETosis at a high concentration or density of immobilized human polyvalent immunoglobulins and thus at low ratio of neutrophils:human polyvalent immunoglobulins.

In some embodiments, a low ratio of neutrophils:human polyvalent immunoglobulins corresponds for example:
- to a culture of neutrophils at a concentration ranging from about 1x10⁵ to about 1x10⁷ cells/mL of culture medium, preferably from about 5×10⁵ to about 5x10⁶ cells/mL of culture medium, more preferably at a concentration of about 1×10⁶ cells/mL of culture medium; and
- to a solid support coated with a coating solution comprising human polyvalent immunoglobulins at a concentration ranging from about 50 µg/mL to about 120 µg/mL, preferably from about 75 µg/mL to about 110 µg/mL, more preferably at a concentration of about 100 µg/mL.

Another object of the present invention is a method for treating a subject susceptible to NET formation and/or NETosis, said method comprising:
a) identifying a subject susceptible to NET formation and/or NETosis by contacting an *in vitro* culture of neutrophils previously obtained from the subject with human polyvalent immunoglobulins immobilized on a solid support, thereby inducing NET formation and/or NETosis, as described above;
b) treating the subject susceptible to NET formation and/or NETosis by administering to the subject an inhibitor of NET formation and/or NETosis.

Examples of inhibitors of NET formation and/or NETosis include disulfiram; peptidyl arginine deiminase 4 (PAD4) inhibitors, such as Cl-amidine; neutrophil elastase (NE) inhibitors, such as sivelestat (also known as elaspol), alvelestat; DNases, such as DNase I; and anti-citrullinated protein antibodies (ACP) also known as therapeutic anti-citrullinated protein antibody (tACPA), such as CIT-013.

In some embodiments, the step of identifying a subject susceptible to NET formation and/or NETosis further comprises determining the ratio of neutrophils:human polyvalent immunoglobulins required to induce maximal NET formation and/or NETosis in the *in vitro* culture of neutrophils previously obtained from the subject. For example, the ratio of neutrophils:human polyvalent immunoglobulins required to induce maximal NET formation and/or NETosis may be determined by contacting *in vitro* cultures of a given concentration (*e*.*g*., about 1x10⁶ cells/mL of culture medium) of neutrophils previously obtained from the subject with increasing densities of human polyvalent immunoglobulins immobilized on a solid support.

Indeed, in some embodiments, a subject susceptible to NET formation and/or NETosis is identified based on the ratio of neutrophils:human polyvalent immunoglobulins required to induce maximal NET formation and/or NETosis in the *in vitro* culture of neutrophils previously obtained from the subject.

In some embodiments, a subject susceptible to NET formation and/or NETosis is a subject identified as a high responder or as an intermediate responder, as described above. In some embodiments, a subject susceptible to NET formation and/or NETosis is a subject identified as a high responder as described above.

The present invention further relates to an *in vitro* method for predicting the response of a subject to a modulator of NET formation and/or NETosis, comprising:
a) contacting an *in vitro* culture of neutrophils previously obtained from a subject with a modulator of NET formation and/or NETosis;
b) inducing NET formation and/or NETosis by contacting the *in vitro* culture of neutrophils with human polyvalent immunoglobulins immobilized on a solid support as described above; and
c) assessing the effect of the modulator of NET formation and/or NETosis on NET formation and/or NETosis in the *in vitro* culture of neutrophils,
wherein the order of step a) and b) can be inverted.

In some embodiments, the method comprises:
a) contacting an *in vitro* culture of neutrophils previously obtained from a subject with a modulator of NET formation and/or NETosis;
b) inducing NET formation and/or NETosis by contacting the *in vitro* culture of neutrophils with human polyvalent immunoglobulins immobilized on a solid support as described above; and
c) assessing the effect of the modulator of NET formation and/or NETosis on NET formation and/or NETosis in the *in vitro* culture of neutrophils.

In some embodiments, the method comprises:
a) inducing NET formation and/or NETosis by contacting an *in vitro* culture of neutrophils previously obtained from a subject with human polyvalent immunoglobulins immobilized on a solid support as described above;
b) contacting the neutrophils in *in vitro* culture with a modulator of NET formation; and/or NETosis and
c) assessing the effect of the modulator of NET formation and/or NETosis on NET formation and/or NETosis in the *in vitro* culture of neutrophils.

In some embodiments, the modulator of NET formation and/or NETosis is an inhibitor of NET formation and/or NETosis. In some embodiments, the modulator of NET and/or NETosis formation is a stimulator of NET formation and/or NETosis.

In some embodiments, the method comprises quantifying NET formation and/or NETosis induced in the *in vitro* culture of neutrophils in presence of the modulator of NET formation and/or NETosis and comparing said NET formation and/or NETosis to a reference value, thereby assessing the response of the subject to a modulator of NET formation and/or NETosis.

In some embodiments, the reference value is the NET formation and/or NETosis induced in an *in vitro* culture of neutrophils previously obtained from the subject in the absence of the modulator of NET formation and/or NETosis.

In some embodiments, when the NET formation and/or NETosis induced in the *in vitro* culture of neutrophils in presence of the modulator of NET formation and/or NETosis is substantially different compared to the reference value, the subject is considered to be a responder (*i.e*., to respond to the modulator of NET formation and/or NETosis).

In some embodiments, the NET formation and/or NETosis induced in the *in vitro* culture of neutrophils in presence of the modulator of NET formation and/or NETosis is substantially different if it is more than about 1% higher, 2% higher, 3% higher, 4% higher, 5% higher, 6% higher, 7% higher, 8% higher, 9% higher, 10% higher, 15% higher, 20% higher, 25% higher, 30% higher, 35 % higher, 40% higher, 45% higher, or 50% higher, or more than the reference value; or if it is more than about 1% lower, 2% lower, 3% lower, 4% lower, 5% lower, 6% lower, 7% lower, 8% lower, 9% lower, 10% lower, 15% lower, 20% lower, 25% lower, 30% lower, 35% lower, 40% lower, 45% lower, or 50% lower, or more than the reference value. In one embodiment, the NET formation and/or NETosis induced in the *in vitro* culture of neutrophils in presence of the modulator of NET formation and/or NETosis is substantially different if it is more than about 5% higher or 5% lower than the reference value.

In some embodiments, when the NET formation and/or NETosis induced in the *in vitro* culture of neutrophils in presence of an inhibitor of NET formation and/or NETosis is substantially lower compared to the reference value, the subject is considered to be a responder (*i.e*., to respond to the inhibitor of NET formation and/or NETosis). In some embodiments, the NET formation and/or NETosis induced in the *in vitro* culture of neutrophils in presence of an inhibitor of NET formation and/or NETosis is substantially lower if it is more than about 1% lower, 2% lower, 3% lower, 4% lower, 5% lower, 6% lower, 7% lower, 8% lower, 9% lower, 10% lower, 15% lower, 20% lower, 25% lower, 30% lower, 35% lower, 40% lower, 45% lower, or 50% lower, or more than the reference value. In one embodiment, the NET formation and/or NETosis induced in the *in vitro* culture of neutrophils in presence of an inhibitor of NET formation and/or NETosis is substantially lower if it is more than about 5% lower than the reference value.

In some embodiments, when the NET formation and/or NETosis induced in the *in vitro* culture of neutrophils in presence of a stimulator of NET formation and/or NETosis is substantially higher compared to the reference value, the subject is considered to be a responder (*i.e*., to respond to the stimulator of NET formation and/or NETosis). In some embodiments, the NET formation and/or NETosis induced in the *in vitro* culture of neutrophils in presence of a stimulator of NET formation and/or NETosis is substantially higher if it is more than about 1% higher, 2% higher, 3% higher, 4% higher, 5% higher, 6% higher, 7% higher, 8% higher, 9% higher, 10% higher, 15% higher, 20% higher, 25% higher, 30% higher, 35 % higher, 40% higher, 45% higher, or 50% higher, or more than the reference value. In one embodiment, the NET formation and/or NETosis induced in the *in vitro* culture of neutrophils in presence of a stimulator of NET formation and/or NETosis is substantially higher if it is more than about 5% higher than the reference value.

Another object of the present invention is a method for treating a subject identified as a responder to a modulator of NET formation and/or NETosis, said method comprising:
a) identifying a subject as a responder to a modulator of NET formation and/or NETosis as described above;
b) treating the subject identified as a responder to the modulator of NET formation and/or NETosis by administering to the subject the modulator of NET formation and/or NETosis.

Thus, in some embodiments, identifying a subject as a responder to a modulator of NET formation and/or NETosis comprises:
i. contacting an *in vitro* culture of neutrophils previously obtained from the subject with a modulator of NET formation and/or NETosis;
ii. inducing NET formation and/or NETosis by contacting the *in vitro* culture of neutrophils with human polyvalent immunoglobulins immobilized on a solid support as described above; and
iii. assessing the effect of the modulator of NET formation and/or NETosis on NET formation and/or NETosis in the *in vitro* culture of neutrophils,
wherein the order of step i and ii can be inverted.

The stimulator of NET formation and/or NETosis may be selected from disulfiram; peptidyl arginine deiminase 4 (PAD4) inhibitors, such as Cl-amidine; neutrophil elastase (NE) inhibitors, such as sivelestat (also known as elaspol), alvelestat; DNases, such as DNase I; anti-citrullinated protein antibodies (ACP) also known as therapeutic anti-citrullinated protein antibody (tACPA), such as CIT-013; and sulfasalazine (SSZ).

In some embodiments, the method comprises quantifying NET formation and/or NETosis induced in the *in vitro* culture of neutrophils in presence of the modulator of NET formation and/or NETosis and comparing said NET formation and/or NETosis to a reference value, thereby identifying a subject as a responder to a modulator of NET formation and/or NETosis.

In some embodiments, the reference value is the NET formation and/or NETosis induced in an *in vitro* culture of neutrophils previously obtained from the subject in the absence of the modulator of NET formation and/or NETosis.

In some embodiments, when the NET formation and/or NETosis induced in the *in vitro* culture of neutrophils in presence of the modulator of NET formation and/or NETosis is substantially different compared to the reference value, the subject is considered to be a responder (*i.e*., to respond to the modulator of NET formation and/or NETosis).

Another object of the present invention is an *in vitro* method for monitoring the response of a subject to an immunomodulator, comprising:
a) contacting an *in vitro* culture of neutrophils previously obtained from a subject with human polyvalent immunoglobulins immobilized on a solid support, thereby inducing NET formation and/or NETosis as described above, wherein said subject was treated or is being treated with an immunomodulator;
b) quantifying NET formation and/or NETosis induced in the *in vitro* culture of neutrophils; and
c) comparing the NET formation and/or NETosis induced in the *in vitro* culture of neutrophils to a reference value, thereby monitoring the response of the subject to the immunomodulator.

In some embodiments, by "was treated or is being treated with an immunomodulator" it is meant that the subject was administered with or is being administered with an immunomodulator.

In some embodiments, "**immunomodulator**" refers to a substance, in particular a therapeutic drug, that modulates an immune or inflammatory response by increasing (immunostimulant or immunostimulator) or decreasing (immunosuppressant or immunosuppressor) the immune or inflammatory response. For example, an immunostimulant (or immunostimulator) may increase an immune or inflammatory response by inducing activation or increasing activity of immune cells. Conversely, an immunosuppressant (or immunosuppressor)may decrease (or inhibit) an immune or inflammatory response by preventing or inhibiting activation or decreasing activity of immune cells.

In some embodiments, the reference value is the NET formation and/or NETosis induced in an *in vitro* culture of neutrophils previously obtained from the subject before treatment with the immunomodulator. Thus, in other words, in some embodiments, the reference value is the NET formation and/or NETosis induced in an *in vitro* culture of neutrophils previously obtained from the subject before said subject was administered with the immunomodulator.

In some embodiments, when the NET formation and/or NETosis induced in the *in vitro* culture of neutrophils previously obtained from a subject who was treated or is being treated with an immunomodulator is substantially different compared to the reference value, the subject is considered to respond to the immunomodulator.

In some embodiments, the NET formation and/or NETosis induced in the *in vitro* culture of neutrophils previously obtained from a subject who was treated or is being treated with an immunomodulator is substantially different if it is more than about 1% higher, 2% higher, 3% higher, 4% higher, 5% higher, 6% higher, 7% higher, 8% higher, 9% higher, 10% higher, 15% higher, 20% higher, 25% higher, 30% higher, 35 % higher, 40% higher, 45% higher, or 50% higher, or more than the reference value; or if it is more than about 1% lower, 2% lower, 3% lower, 4% lower, 5% lower, 6% lower, 7% lower, 8% lower, 9% lower, 10% lower, 15% lower, 20% lower, 25% lower, 30% lower, 35% lower, 40% lower, 45% lower, or 50% lower, or more than the reference value. In one embodiment, the NET formation and/or NETosis induced in the *in vitro* culture of neutrophils previously obtained from a subject who was treated or is being treated with an immunomodulator is substantially different if it is more than about 5% higher or 5% lower than the reference value.

In some embodiments, when the NET formation and/or NETosis induced in the *in vitro* culture of neutrophils previously obtained from a subject who was treated or is being treated with an immunostimulant (or immunostimulator) is substantially higher compared to the reference value, the subject is considered to respond to the immunostimulant (or immunostimulator).

In some embodiments, the NET formation and/or NETosis induced in the *in vitro* culture of neutrophils previously obtained from a subject who was treated or is being treated with an immunostimulant (or immunostimulator) is substantially higher if it is more than about 1% higher, 2% higher, 3% higher, 4% higher, 5% higher, 6% higher, 7% higher, 8% higher, 9% higher, 10% higher, 15% higher, 20% higher, 25% higher, 30% higher, 35 % higher, 40% higher, 45% higher, or 50% higher, or more than the reference value. In one embodiment, the NET formation and/or NETosis induced in the *in vitro* culture of neutrophils previously obtained from a subject who was treated or is being treated with an immunostimulant (or immunostimulator) is substantially higher if it is more than about 5% higher than the reference value.

In some embodiments, when the NET formation and/or NETosis induced in the *in vitro* culture of neutrophils previously obtained from a subject who was treated or is being treated with an immunosuppressant (immunosuppressor) is substantially lower compared to the reference value, the subject is considered to respond to the immunosuppressant or (immunosuppressor).

In some embodiments, the NET formation and/or NETosis induced in the *in vitro* culture of neutrophils previously obtained from a subject who was treated or is being treated with an immunosuppressant (immunosuppressor) is substantially lower if it is more than about 1% lower, 2% lower, 3% lower, 4% lower, 5% lower, 6% lower, 7% lower, 8% lower, 9% lower, 10% lower, 15% lower, 20% lower, 25% lower, 30% lower, 35% lower, 40% lower, 45% lower, or 50% lower, or more than the reference value. In one embodiment, the NET formation and/or NETosis induced in the *in vitro* culture of neutrophils previously obtained from a subject who was treated or is being treated with an immunosuppressant (immunosuppressor) is substantially lower if it is more than about 5% lower than the reference value.

In some embodiments, the subject is suffering from a disease selected from an inflammatory disease, a thromboembolic disease, a solid cancer and a fibrosis. In some embodiments, said disease is a neutrophil-associated disease or condition. In some embodiments, the neutrophil-associated disease or condition is a disease or condition to which NET formation and/or NETosis may contribute.

Thus, in some embodiments, the neutrophil-associated disease or condition, in particular a disease or condition to which NET formation and/or NETosis may contribute, is an inflammatory disease, a thromboembolic diseases, a solid cancer, or a fibrosis.

In some embodiments, the inflammatory disease is an autoinflammatory disease or an autoimmune disease. In some embodiments, the inflammatory disease is a chronic inflammatory disease. In some embodiments, the inflammatory disease is an acute inflammatory disease.

In some embodiments, the inflammatory disease is vasculitis. In some embodiments, the inflammatory disease is anti-neutrophil cytoplasmic autoantibody (ANCA)-associated vasculitis or ANCA vasculitis.

In some embodiments, the cancer is a solid cancer. Examples of solid cancers include breast cancer, lung cancer, pancreatic cancer, colon cancer, kidney cancer, prostate cancer.

In some embodiments, the fibrosis is cystic fibrosis. In some embodiments, the fibrosis is a fibrosis associated with a chronic inflammatory disease or a complication of a chronic inflammatory disease. Examples of such fibroses include liver fibrosis, lung fibrosis, kidney fibrosis or renal fibrosis in chronic kidney disease.

In some embodiments, the neutrophils-associated disease or condition, in particular a disease or condition to which NET formation and/or NETosis may contribute, is selected from the group comprising or consisting of vasculitis, in particular ANCA vasculitis; asthma; rheumatoid arthritis (RA); chronic obstructive pulmonary disease (COPD); systemic lupus erythematous (SLE); systemic sclerosis; adverse cardiovascular events such as myocardial infarction; ischemic stroke; atherosclerosis; venous thrombosis, cancer, in particular solid cancer; fibrosis, for example cystic fibrosis; sepsis; gout; and Alzheimer's disease.

### BRIEF DESCRIPTION OF THE DRAWINGS

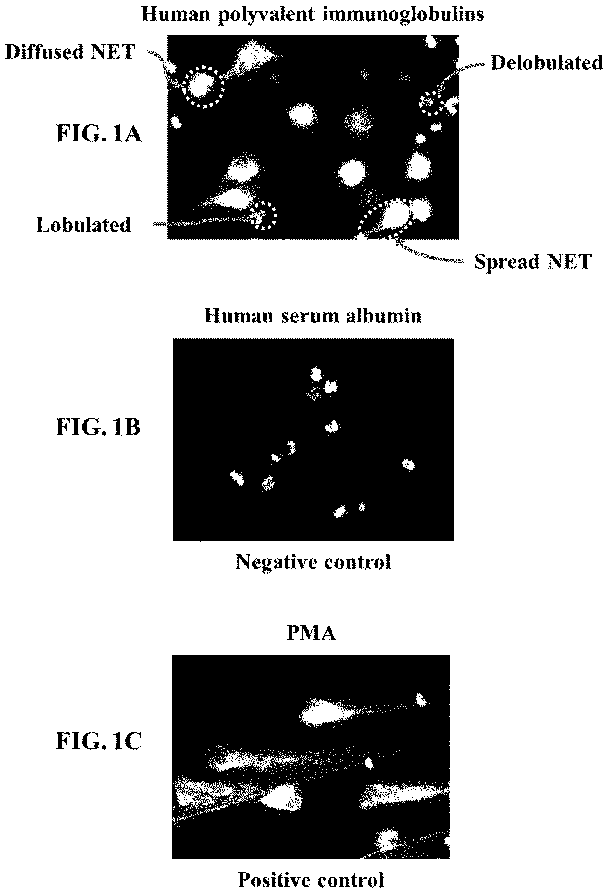

**Figure 1** is a combination of images obtained with fluorescence microscopy showing NET formation by freshly isolated human peripheral blood neutrophils cultured *in vitro* in presence of the cell-impermeable nucleic acid dye Sytox Green. **Figure 1A** shows human neutrophils cultured in plates coated with human polyvalent immunoglobulins (obtained with a coating solution comprising human polyvalent Ig at a concentration of 100 µg/mL). **Figure 1B** shows human neutrophils cultured in plates coated with human serum albumin or HSA (obtained with a coating solution comprising HSA at a concentration of 100 µg/mL), used as negative control. **Figure 1C** shows human neutrophils cultured with PMA (25 nM), used as positive control.

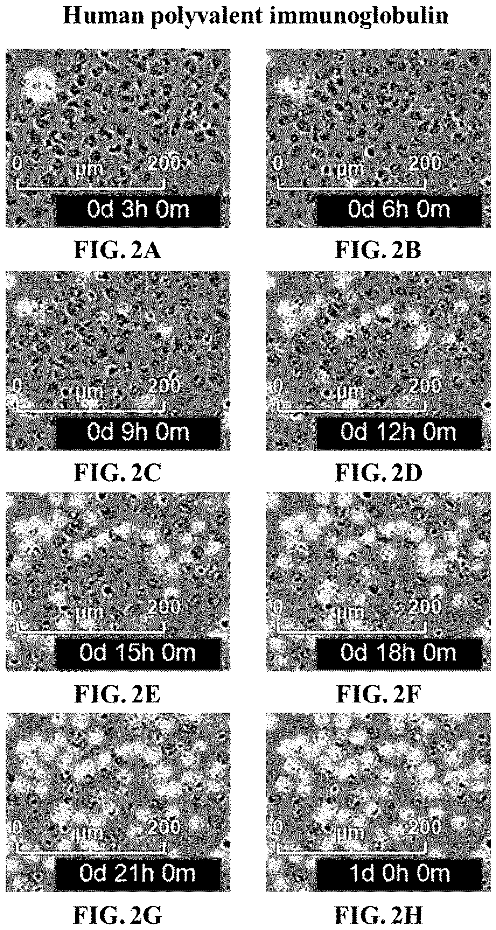

**Figure 2** is a combination of images obtained with live cell fluorescent microscopy showing NET formation over a 24-hour culture period by freshly isolated human peripheral blood neutrophils cultured *in vitro* in plates coated with human polyvalent Ig (obtained with a coating solution comprising human polyvalent Ig at a concentration of 100 µg/mL). NET formation is shown after 3-, 6-, 9-, 12-, 15-, 18-, 21- and 24-hour culture in presence of the cell-impermeable nucleic acid dye Sytox Green. **Figure 2A** shows 3-hour culture. **Figure 2B** shows 6-hour culture. **Figure 2C** shows 9-hour culture. **Figure 2D** shows 12-hour culture. **Figure 2E** shows 15-hour culture. **Figure 2F** shows 18-hour culture. **Figure 2G** shows 21-hour culture. **Figure 2H** shows 24-hour (1 day) culture.

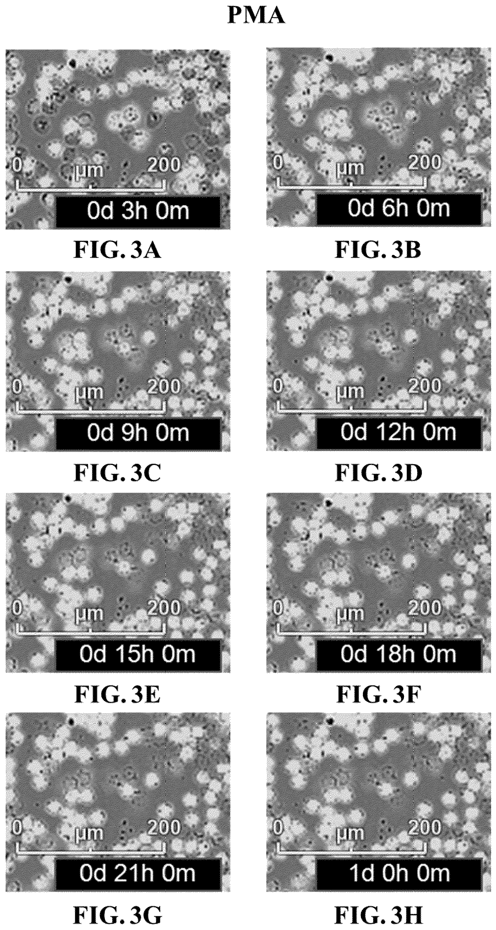

**Figure 3** is a combination of images obtained with live cell fluorescent microscopy showing NET formation over a 24-hour culture period by freshly isolated human peripheral blood neutrophils cultured *in vitro* with PMA (25 nM). NET formation is shown after 3-, 6-, 9-, 12-, 15-, 18-, 21- and 24-hour culture in presence of the cell-impermeable nucleic acid dye Sytox Green. **Figure 3A** shows 3-hour culture. **Figure 3B** shows 6-hour culture. **Figure 3C** shows 9-hour culture. **Figure 3D** shows 12-hour culture. **Figure 3E** shows 15-hour culture. **Figure 3F** shows 18-hour culture. **Figure 3G** shows 21-hour culture. **Figure 3H** shows 24-hour (1 day) culture.

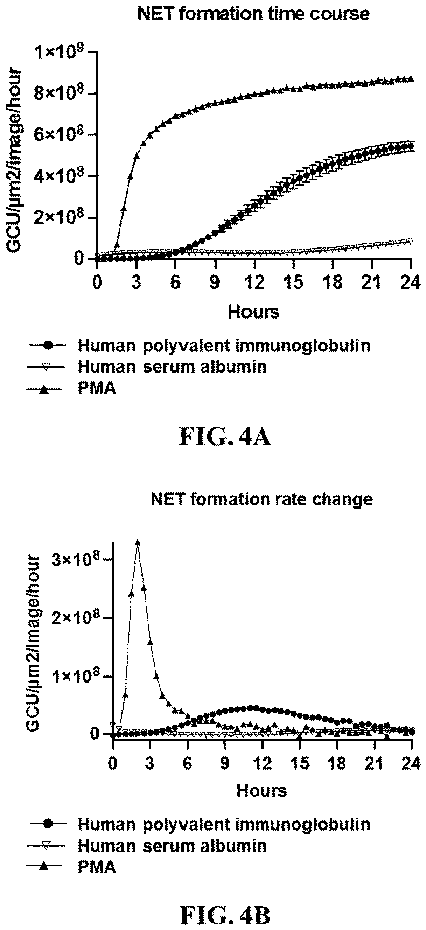

**Figure 4** is a combination of graphs showing the NET formation time-course (**Figure 4A**) and the NET formation rate change (**Figure 4B**) over a 24-hour culture period of freshly isolated human peripheral blood neutrophils cultured *in vitro* in presence of the cell-impermeable nucleic acid dye Sytox Green, either with plate-bound human polyvalent Ig (obtained with a coating solution comprising human polyvalent Ig at a concentration of 100 µg/mL), plate-bound human serum albumin (negative control, obtained with a coating solution comprising HSA at a concentration of 100 µg/mL), or 25 nM PMA (positive control). NET formation is assessed at 0-, 3-, 6-, 9-, 12-, 15-, 18-, 21-, and 24-hour culture. Data are expressed as integrated fluorescence intensity (green calibrated unit or GCU)/µ²/image/well.

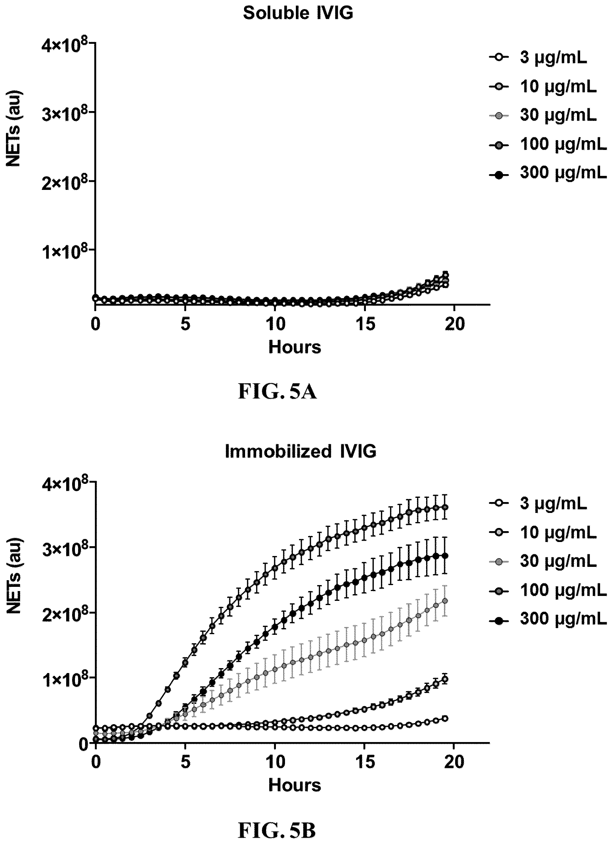

**Figure 5** is a combination of graphs comparing the formation of NETs by freshly isolated human peripheral blood neutrophils cultured *in vitro* in presence of the cell-impermeable nucleic acid dye Sytox Green, either with soluble human polyvalent Ig (soluble IVIG - **Figure 5A**) or with plate-bound human polyvalent Ig (immobilized IVIG - **Figure 5B**). Human polyvalent Ig are used at the indicated concentration (concentration in the coating solution for the plate-bound human polyvalent Ig). Data correspond to integrated green fluorescent intensity expressed as GCU/well.

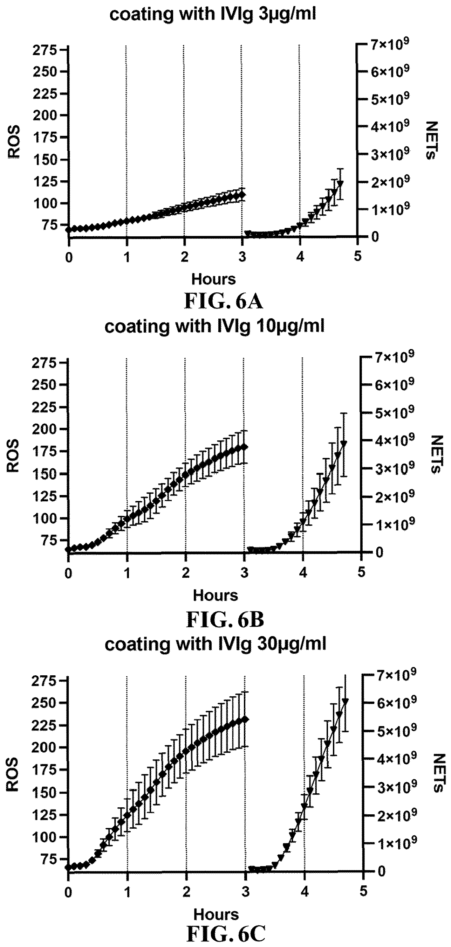

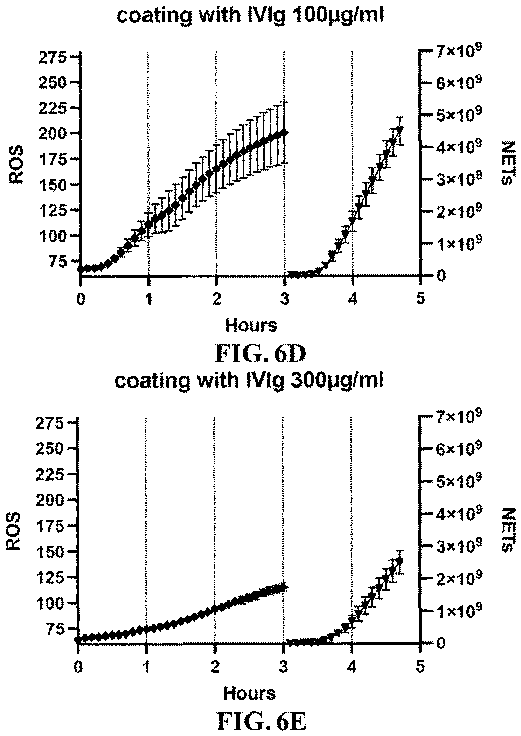

**Figure 6** is a combination of graphs showing the production of ROS (arbitrary units), detected through the use of an oxidative-sensitive fluorescent probe, and the formation of NETs (arbitrary units), detected through the use of the cell-impermeable nucleic acid dye Sytox Green, over a 5-h time period by freshly isolated human peripheral blood neutrophils cultured *in vitro* in plates coated with human polyvalent Ig (or IVIg). Data are expressed as GCU/well. Increasing densities of plate-bound human polyvalent Ig are used, obtained with a coating solution comprising human polyvalent Ig at a concentration ranging from 3 to 300 µg/mL. **Figure 6A** corresponds to deposits of IVIg obtained with a coating solution comprising IVIg at a concentration of 3 µg/mL. **Figure 6B** corresponds to deposits of IVIg obtained with a coating solution comprising IVIg at a concentration of 10 µg/mL. **Figure 6C** corresponds to deposits of IVIg obtained with a coating solution comprising IVIg at a concentration of 30 µg/mL. **Figure 6D** corresponds to deposits of IVIg obtained with a coating solution comprising IVIg at a concentration of 100 µg/mL. **Figure 6E** corresponds to deposits of IVIg obtained with a coating solution comprising IVIg at a concentration of 300 µg/mL.

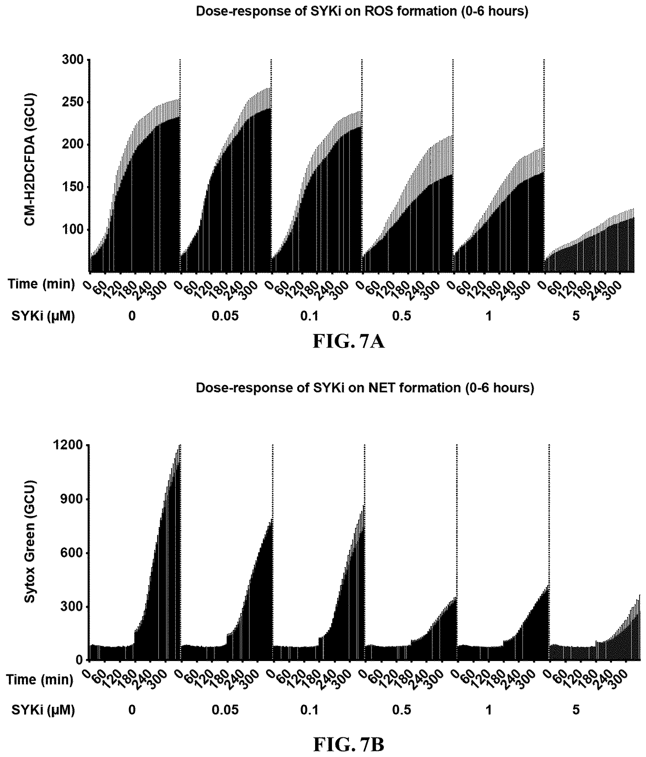

**Figure** 7 is a combination of graphs showing the production of ROS (CM-H₂DCFDA - **Figure 7A**) and the formation of NETs (Sytox Green - **Figure 7B**) over a period of 6 hours (0-360 min) by freshly isolated human peripheral blood neutrophils cultured *in vitro* in plates coated with human polyvalent Ig (obtained with a coating solution comprising human polyvalent Ig at a concentration of 100 µg/mL) in the presence of different concentrations of the SYK inhibitor (or SYKi) BAY-61-3606 (0, 0.05, 0.1, 0.5, 1, 5 µM, as indicated). Data are expressed as GCU/well.

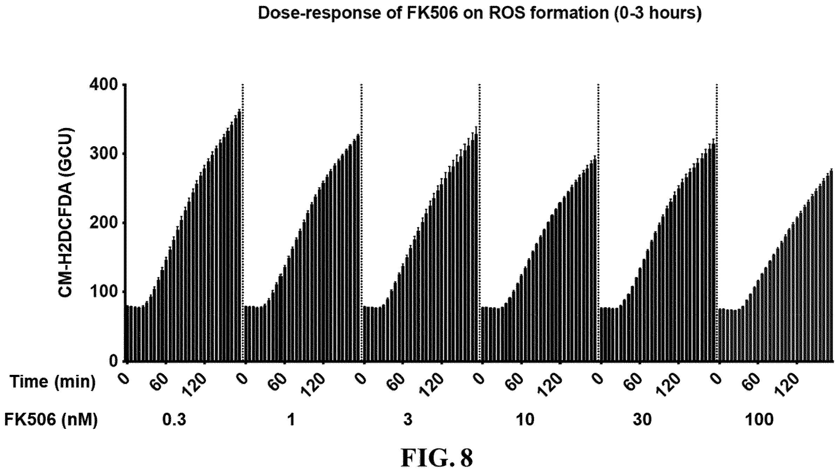

**Figure 8** is a graph showing the production of ROS (CM-H₂DCFDA) over a period of 3 hours (0-180 minutes) by freshly isolated human peripheral blood neutrophils cultured *in vitro* in plates coated with human polyvalent Ig (obtained with a coating solution comprising human polyvalent Ig at a concentration of 100 µg/mL) in the presence of different concentrations of the calcineurin inhibitor FK506, also known as tacrolimus (0.3, 1, 3, 10, 30, 100 nM, as indicated). Data are expressed as GCU/well.

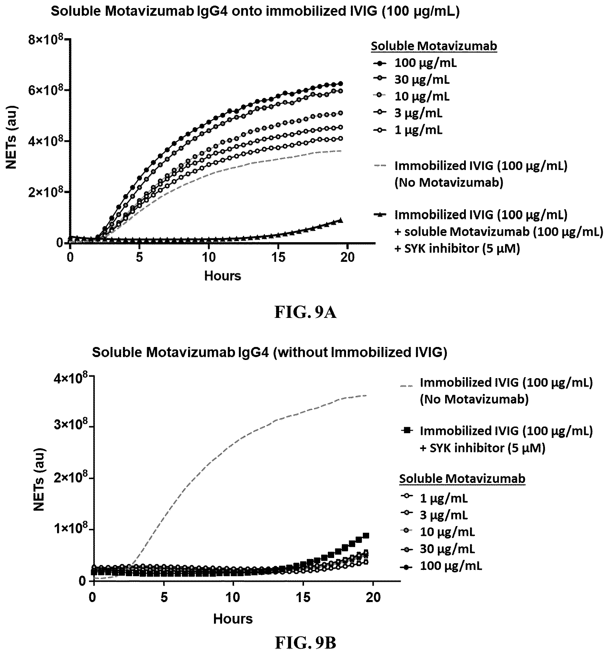

**Figure 9** is a combination of graphs comparing the formation of NETs by freshly isolated human peripheral blood neutrophils cultured *in vitro* in presence of the cell-impermeable nucleic acid dye Sytox Green with soluble motavizumab IgG4 (used at increased concentration ranging from 1 to 100 µg/mL, as indicated) either with plate-bound human polyvalent Ig (immobilized IVIG - **Figure 9A**) or without plate-bound human polyvalent Ig (without immobilized IVIG - **Figure 9B**). The SYK inhibitor BAY-61-3606 (5 µM) is used as a control (SYKi) in combination with immobilized human polyvalent Ig (**Figure 9B**) or in combination with both immobilized human polyvalent Ig and 100 µg/mL of soluble motavizumab IgG4 (**Figure 9A**). When present, deposits of human polyvalent Ig are obtained with a coating solution comprising human polyvalent Ig at a concentration of 100 µg/mL. Data are expressed as GCU/well.

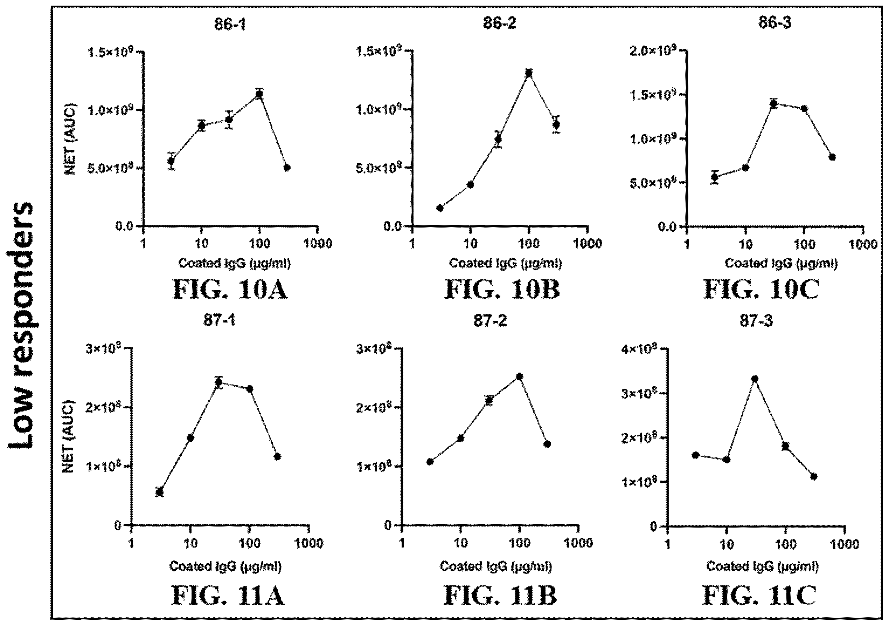

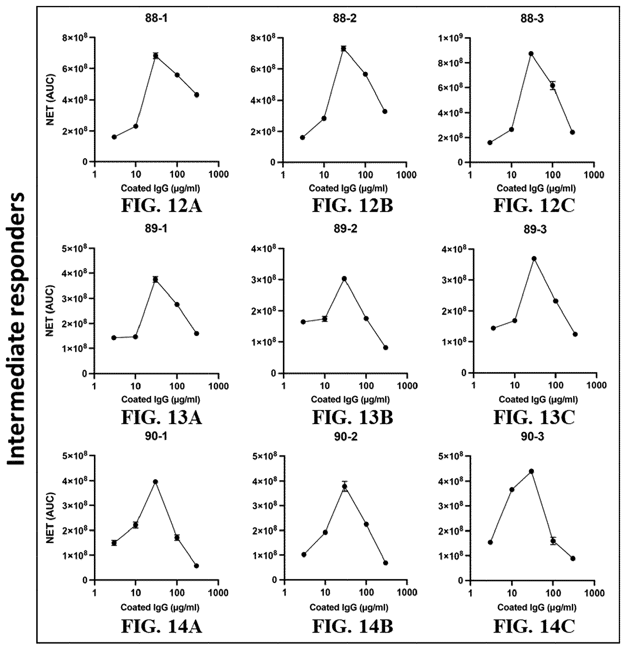

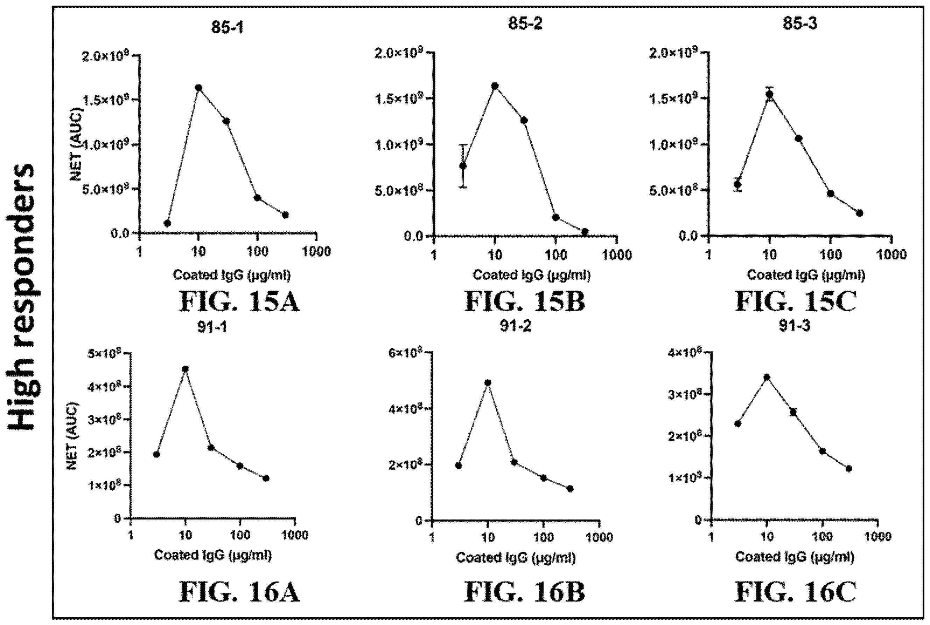

**Figures 10-16** are combinations of graphs showing the formation of NETs (arbitrary units - AUC) by human neutrophils freshly isolated from samples of peripheral blood of 7 healthy individuals (referred to as 85, 86, 87, 88, 89, 90, and 91) as a function of the density of human polyvalent Ig deposits. For each individual, neutrophils were isolated from samples of peripheral blood obtained at three independent time points (time points 1, 2, and 3), separated by a least two weeks. For each individual, isolated neutrophils were cultured in presence of the cell-impermeable nucleic acid dye Sytox Green with increasing densities of human polyvalent Ig obtained with a coating solution comprising human polyvalent Ig at a concentration of 3, 10, 30, 100 or 300 µg/mL. **Figure 10** shows donor 86 at time point 1 (**Figure 10A**), time point 2 (**Figure 10B**) and time point 3 (**Figure 10C**). **Figure 11** shows donor 87 at time point 1 (**Figure 11A**), time point 2 (**Figure 11B**) and time point 3 (**Figure 11C**). **Figure 12** shows donor 88 at time point 1 (**Figure 12A**), time point 2 (**Figure 12B**) and time point 3 (**Figure 12C**). **Figure 13** shows donor 89 at time point 1 (**Figure 13A**), time point 2 (**Figure 13B**) and time point 3 (**Figure 13C**). **Figure 14** shows donor 90 at time point 1 (**Figure 14A**), time point 2 (**Figure 14B**) and time point 3 (**Figure 14C**). **Figure 15** shows donor 85 at time point 1 (**Figure 15A**), time point 2 (**Figure 15B**) and time point 3 (**Figure 15C**). **Figure 16** shows donor 91 at time point 1 (**Figure 16A**), time point 2 (**Figure 16B**) and time point 3 (**Figure 16C**).

### EXAMPLES

The present invention is further illustrated by the following examples.

### Example 1: Evaluation of NET formation by human neutrophils interacting with plate-bound human polyvalent immunoglobulins

### Materials and Methods

### Reagents

Human polyvalent immunoglobulins (or human polyvalent Ig) also referred to as intravenous immunoglobulin or IVIg (Privigen, CSL Behring LLC) or human serum albumin or HSA (Sigma Aldrich - reference: H5667), used as negative control, diluted in coating solution (DPBS Gibco - reference: 14190) were allowed to adsorb for 2 hours at room temperature on high-binding, sterile 96-well plates suitable for fluorescence microscopy (Greiner Bio-One, Frickenhausen, Germany). Unless indicated otherwise, deposits of human polyvalent Ig were obtained with a coating solution (DPBS no calcium, no magnesium (ThermoFisher - reference: 14190) consisting of KCl 2.67 mM, KH₂PO₄ 1.47 mM, NaCl 137.9 mM, Na₂HPO₄-7H₂O 8.06 mM) comprising human polyvalent Ig at a concentration of 100 µg/mL. Similarly, unless indicated otherwise, deposits of HSA were obtained with a coating solution comprising HSA at a concentration of 100 µg/mL. Phorbol-12-myristate-13-acetate or PMA (Sigma Aldrich - reference: P8139) was used as positive control. PMA was added to the culture medium at a final concentration of 25 nM. In some experiments (see **Figure 5A**), human polyvalent Ig were added in the culture medium at a final concentration ranging from 3 to 300 µg/mL.

### Cells and cell culture

Neutrophils were isolated from the peripheral blood of healthy human donors by negative magnetic bead selection using a commercially available kit (STEMCELL Technologies - reference: 19666). Isolated neutrophils were then distributed in 96-well plates (Greiner Bio-One, Frickenhausen, Germany), in particular in 96-well plates coated with human polyvalent Ig or HSA as described above, at a concentration of 7.5×10⁴ cells per well, corresponding to 10⁶ cells/mL of culture medium. Neutrophils were then cultured in HBSS with calcium and magnesium (Gibco - reference: 24020-091) under standard conditions (*i.e*., humidified incubator 37°C and 5% CO₂).

### NET detection: fixed neutrophils

After 4 hours of culture, neutrophils were fixed with paraformaldehyde or PFA (2%), permeabilized with Triton X100 (0.1%) and stained with 1 µM Sytox Green (Invitrogen - reference: S7020 ), which specifically stains nucleic acids.

Images were acquired by fluorescence microscopy in the green channel. NET induction was identified by the nuclear morphology of the visualized neutrophils.

### NET detection: live imaging

Freshly isolated human neutrophils were distributed in 96-well plates coated with human polyvalent Ig (obtained with a coating solution comprising human polyvalent Ig at a concentration of 100 µg/mL) or HSA (obtained with a coating solution comprising HSA at a concentration of 100 µg/mL) and cultured for 24 hours in culture medium containing the fluorescent, cell-impermeant, nucleic acid dye Sytox Green (1 µM). Alternatively, as a positive control, freshly isolated human neutrophils were cultured in a 96-well plate in culture medium comprising PMA (25 nM) and Sytox Green (1 µM).

Time-lapse images were acquired by live cell fluorescent microscopy in 9 spots from each well (conditions are run in triplicates). Time-course quantitative measure of NET formation was performed by computer-assisted image analysis. data are expressed as integrated fluorescence intensity (GCU)/µ²/image/well.

### Results

### NET formation is induced in vitro by human blood neutrophils interacting with plate-bound deposits of human polyvalent Ig

Formation of NETs was assessed in 3 conditions: neutrophils cultured in the presence of plate-bound human polyvalent immunoglobulins (IVIG), neutrophils cultured in the presence of plate-bound HSA (negative control), and neutrophils cultured in the presence of PMA, a known inducer of NETs. Different morphologies of neutrophils undergoing NETosis were observed in wells coated with human polyvalent immunoglobulins: neutrophils with a lobulated nucleus (absence of NET formation), neutrophils with a delobulated nucleus, as well as diffused NETs and spread NETs (**Figure 1A**). By contrast, neutrophils all appeared lobulated (absence of NET formation) in the negative control wells coated with human serum albumin **(****Figure 1B**). In positive control wells (presence of PMA), most neutrophils displayed signs of NETosis, *i.e*., diffused and spread NETs (**Figure 1C**).

These results demonstrate the ability of plate-bound human polyvalent Ig to induce NET formation from isolated human neutrophils.

### NET formation induced in vitro by the interaction of neutrophils with immobilized human polyvalent Ig is slow and progressive

To better characterize the formation of NETs, live imaging was carried out. Three conditions were assessed: neutrophils cultured in the presence of plate-bound human polyvalent Ig (IVIg), neutrophils cultured in the presence of plate-bound HSA (negative control), and neutrophils cultured in the presence of PMA, a known inducer of NETs.

As shown on **Figures 2A-H**, in the presence of plate-bound human polyvalent Ig, progressive NET induction was visible by the immediate staining of extruded nucleic acid, appearing as large fluorescent spots (diffused NETs) at sites of individual neutrophil-Ig interaction at the bottom of the wells (phase contrast) from 3-hour culture to 15-hour culture. By contrast, in the presence of PMA, NET induction was visible on almost all neutrophils from 3-hour culture (see Figure 3A-H).

Quantitative assessment of NET formation over time was also performed by computer-assisted image analysis (see **Figure 4****).** Data reflecting NET formation are expressed as integrated fluorescence intensity (GCU)/µ²/image/well. As shown by the time course of NET formation, the process induced by the interaction of neutrophils with coated human polyvalent Ig is slow and affects discrete cells (**Figures 2A-H** and **Figure 4A**), likely reflecting their interaction with immunoglobulin deposits on the bottom of the wells. By contrast, the presence of PMA in the culture medium drives the simultaneous formation of NETs from a great number of neutrophils, within as soon as 3 hours of culture (**Figures 3A-H** and **Figure 4A**).

Furthermore, as shown by the NET formation rate change, as opposed to PMA which causes NET formation from all neutrophils at once, coated human polyvalent Ig trigger a progressive and cumulative response (**Figure 4B**).

### NET formation is induced in vitro by human polyvalent Ig only if the latter are immobilized onto a solid support

To verify the importance of human polyvalent Ig immobilization, formation of NETs was assessed with neutrophils cultured in the presence of plate-bound human polyvalent Ig and with neutrophils cultured in the presence of human polyvalent Ig added to the culture medium. In both conditions, freshly isolated human peripheral blood neutrophils (7.5×10⁴/well, corresponding to 1×10⁶/mL of culture medium) were cultured in the presence of Sytox Green, with increasing amounts of human polyvalent Ig. Quantitative assessment of NET formation over time was performed by computer-assisted image analysis (arbitrary units, data correspond to integrated green fluorescent intensity expressed as GCU/well).

As shown on **Figure 5A**, the use of soluble human polyvalent Ig does not elicit the formation of NETs at any of the tested concentrations. Strikingly, as shown on **Figure 5B**, NET formation is clearly elicited by the interaction of neutrophils with immobilized polyvalent human Ig, in a dose-dependent manner. Indeed, the occurrence of NET formation appears to be a function of the ratio neutrophils:immunoglobulins. As shown on **Figure 5B**, NET formation starts earlier and increases progressively from 3 µg/mL and up to 100 µg/mL of human polyvalent immunoglobulins (concentration in the coating solution used to prepare the coated culture plates). Of note, NET formation with a concentration of 300 µg/mL of human polyvalent Ig (concentration in the coating solution used to prepare the coated culture plates) starts later and reaches a lower maximal level as compared to the maximal response obtained with a concentration of 100 µg/mL of human polyvalent Ig.

### Example 2: Formation of NETs induced by human polyvalent immunoglobulins faithfully mimics in vivo SYK-dependent NET formation

### Materials and Methods

### Reagents

High-binding, sterile 96-well plates (Greiner Bio-One, Frickenhausen, Germany) were coated with human polyvalent Ig as described in Example 1, using a coating solution comprising human polyvalent Ig at a concentration ranging from 3 to 300 µg/mL, as indicated. If no indication is given, deposits of human polyvalent Ig were obtained with a coating solution comprising human polyvalent Ig at a concentration of 100 µg/mL.

Where indicated, the SYK inhibitor BAY-61-3606, obtained from Selleckchem (reference: S7006), was added to the culture medium at a final concentration ranging from 0.05 to 5 µM (0.05, 0.1, 0.5, 1, 5 µM, as indicated). Where indicated, the calcineurin inhibitor FK506 (also known as tacrolimus), obtained from ASTELLAS PHARMA (PROGRAF^{™} 5 mg/ml), was added to the culture medium at a final concentration ranging from 0.3 to 100 nM (0.3, 1, 3, 10, 30, 100 nM, as indicated). Where indicated, the humanized monoclonal antibody motavizumab directed against the respiratory syncytial virus (RSV), produced as a human IgG4 (by Evitria, Zurich, Switzerland), was added to the culture medium at a final concentration ranging from 1 to 100 µg/mL (1, 3, 10, 30, 100 µg/mL, as indicated). Human IgG4 can engage FcRI (CD64) and enhance the activation of human neutrophils triggered by immobilized immunoglobulins, which is mediated by FcRIIa and FcRIIIb, by the engagement of an additional signaling pathway.

### Cells and cell culture

Human peripheral blood neutrophils were isolated and cultured as described in Example 1. In particular, freshly isolated peripheral blood neutrophils were distributed in coated plate as described in Example 1 (7.5×10⁴ cells per well, corresponding to 10⁶ cells/mL of culture medium) and cultured for the indicated time.

### Detection

The fluorescent, cell-impermeant, nucleic acid dye Sytox Green was added to the culture medium at a concentration of 1 µM to detect NET formation. ROS (reactive oxygen species) formation was identified by the use of a fluorescent oxidative-sensitive probe (CM-H₂DCFDA, ThermoFisher Scientific - reference: C6827). Cells were preincubated for 30 minutes at 37°C with 5 µM CM-H₂DCFDA in HBSS no calcium, no magnesium. Excess CM-H₂DCFDA was eliminated by cell centrifugation. The labeled cell pellet was resuspended in the culture medium (HBSS with calcium and magnesium).

Time-lapse images were acquired by live cell fluorescent microscopy. Quantitative time-course measure of ROS and NET formation was performed by computer-assisted image analysis (data are expressed as GCU/well).

### Results

### NET formation induced in vitro by human polyvalent Ig is preceded by ROS formation and depends on the density of the human polyvalent Ig deposits

To characterize the underlying mechanism and signaling pathway responsible for the formation of NETs when neutrophils are cultured with plate-bound human polyvalent Ig, the formation of ROS was detected over time, in parallel with the formation of NETs.

**Figures 6A-E** show the formation of ROS over a 3-hour period starting from the beginning of the culture of neutrophils in a 96-well plate coated with human polyvalent immunoglobulins, and the production of NETs over a 3-hour period starting 3 hours after the beginning of the culture of neutrophils. Several densities of deposits of human polyvalent immunoglobulins were used (obtained with a coating solution comprising human polyvalent immunoglobulins at a concentration ranging from 3 to 300 µg/mL). As shown on the left part of **Figures 6A-E**, ROS formation is rapidly induced, with deposits of human polyvalent immunoglobulins obtained with a coating solution comprising human polyvalent immunoglobulins at a concentration as low as 3 µg/mL (**Figure 6A**). ROS formation increases in a dose-dependent manner with deposits of human polyvalent immunoglobulins obtained with a coating solution comprising human polyvalent immunoglobulins at a concentration of 10 µg/mL (**Figure 6B**), 30 µg/mL (**Figure 6C**), 100 µg/mL (**Figure 6D**). ROS formation then decreases when using deposits of human polyvalent immunoglobulins obtained with a coating solution comprising human polyvalent immunoglobulins at a concentration of 300 µg/mL (**Figure 6E**). The right part of **Figure 6A-E** shows that the formation of NETs occurs after the production of ROS, in a dose dependent-manner. NETs formation increases with deposits of human polyvalent immunoglobulins obtained with a coating solution comprising human polyvalent immunoglobulins at a concentration of 3 µg/mL (**Figure 6A**), 10 µg/mL (**Figure 6B**), 30 µg/mL (**Figure 6C**), and then decreases with deposits of human polyvalent immunoglobulins obtained with a coating solution comprising human polyvalent immunoglobulins at a concentration of 100 µg/mL (**Figure 6D**) and 300 µg/mL (**Figure 6E**).

These results demonstrate that plate-bound human polyvalent Ig are able to induce NET formation, as well as ROS formation, in a dose-dependent manner. The production of ROS precedes the formation of NETs, as expected given the widely described role of ROS production in NET formation (see for example van der Linden M et al. Sci Rep. 2017 Jul 26;7(1):6529).

### NET formation induced in vitro by human polyvalent Ig relies on a SYK mediated signaling pathway

Next, the implication of SYK signaling was investigated by detecting the production of ROS and the formation of NETs over a period of 6 hours (0-360 minutes) when neutrophils are cultured with plate-bound human polyvalent Ig in the absence and in the presence of the SYK inhibitor BAY-61-3606 (also referred herein as SYKi).

The SYK inhibitor BAY-61-3606, obtained from Selleckchem, was thus added at a final concentration ranging from 0 to 5 µM, as indicated, to cultures of freshly isolated human peripheral blood neutrophils in 96-well plates coated with human polyvalent Ig. The coated plates were obtained with a coating solution comprising human polyvalent Ig at a concentration of 100 µg/mL.

As shown on **Figure 7**, both ROS production (**Figure 7A**) and NET (**Figure 7B**) formation are decreased by the SYK inhibitor BAY-61-3606 in a dose-dependent manner.

These results demonstrate that both ROS production and NET formation induced by immobilized human polyvalent Ig are dependent on the SYK signaling downstream of the Fc receptors expressed at the surface of neutrophils. Moreover, these results demonstrate that immobilized human polyvalent Ig as described herein constitute a physiological inducer of NET formation, which acts via signaling pathways that are involved in the formation of NETs *in vivo.*

The use of immobilized human polyvalent Ig as described herein thus allows to induce NET formation that is susceptible to be modulated by drugs acting on the signaling pathways that are involved in the formation of NETs *in vivo.* The use of immobilized human polyvalent Ig as described herein provides a physiologically relevant assay to reproduce NET formation triggered *in vivo* and can be used to identify, screen and/or assess drugs which may act as immunomodulators, for example to inhibit neutrophil activation and NET formation in the treatment of diseases, such as autoimmune diseases.

### ROS production induced in vitro by human polyvalent Ig is calcium-dependent

Finally, the importance of calcium signaling was investigated by detecting the production of ROS over a period of 3 hours (0-180 minutes) when neutrophils are cultured with plate-bound human polyvalent Ig in the absence and in the presence of the calcineurin inhibitor FK506 (also known as tacrolimus).

The calcineurin inhibitor FK506 (also known as tacrolimus) was thus added at a final concentration ranging from 0.3 to 100 nM, as indicated, to cultures of freshly isolated human peripheral blood neutrophils in 96-well plates coated with human polyvalent Ig. The coated plates were obtained with a coating solution comprising human polyvalent Ig at a concentration of 100 µg/mL.

As shown on **Figure 8**, ROS production was modestly but consistently reduced in response to the calcineurin inhibitor, in a dose-dependent manner. These results demonstrate that ROS production is blunted, in a dose-dependent way, by the blockade of calcineurin signaling downstream SYK, itself downstream of the Fc receptors expressed at the surface of neutrophils. These results thus confirm that immobilized human polyvalent Ig as described herein constitute a physiological inducer of NET formation, which may be helpful to identify, screen and/or assess drugs targeting the different actors involved in the physiological signaling pathways responsible for NET formation *in vivo.*

### NET formation induced in vitro by human polyvalent Ig can be modulated with motavizumab, a monoclonal IgG4

The potentiality for modulation of NET formation was investigated by detecting the formation of NETs over time when neutrophils are cultured with plate-bound human polyvalent Ig in the absence and in the presence of the monoclonal IgG4 motavizumab which can engage FcRI (CD64), thus driving a supplementary SYK-dependent signal, in addition to the one induced by the immobilized human polyvalent Ig (which is mediated by FcRIIa and FcRIIIb).

The monoclonal IgG4 motavizumab was added at a final concentration ranging from 0 to 100 µg/mL, as indicated, to cultures of freshly isolated human peripheral blood neutrophils in 96-well plates coated with human polyvalent Ig. The coated plates were obtained with a coating solution comprising human polyvalent Ig at a concentration of 100 µg/mL, As a control, the SYK inhibitor BAY-61-3606 was used at a concentration of 5 µM (in the concomitant presence of 100 µg/mL motavizumab and immobilized human polyvalent Ig).

As shown on **Figure 9A**, motavizumab added at increasing concentration in the culture medium drives a proportional increase in the formation of NETs as compared to the control (coated IVIG at 100µg/ml without motavizumab). Of note, the use of the SYK inhibitor BAY-61-3606 completely abolishes the NET formation elicited by the concomitant presence of 100 µg/mL motavizumab and immobilized human polyvalent Ig.

These results thus demonstrate that formation of NETs elicited by immobilized human polyvalent immunoglobulins can be further enhanced by concomitant signaling stimuli.

Next, NET formation was detected after addition of the monoclonal IgG4 motavizumab at a final concentration ranging from 0 to 100 µg/mL, as indicated, to cultures of freshly isolated human peripheral blood neutrophils in uncoated 96-well plates (*i.e*., in the absence of immobilized human polyvalent Ig). As a positive control, NET formation was detected in freshly isolated human peripheral blood neutrophils cultured in 96-well plates coated with human polyvalent Ig. NET formation was also detected in freshly isolated human peripheral blood neutrophils cultured in the presence of 5 µM of the SYK inhibitor BAY-61-3606 in 96-well plates coated with human polyvalent Ig. The coated plates were obtained with a coating solution comprising human polyvalent Ig at a concentration of 100 µg/mL.

As shown on **Figure 9B**, in the absence of immobilized immunoglobulins, the addition of motavizumab at increasing concentration in the culture medium is not able to drive the formation of NETs. By contrast, the formation of NETs is readily detectable when freshly isolated human peripheral blood neutrophils are cultured in 96-well plates coated with human polyvalent Ig. Of note, even when the SYK inhibitor BAY-61-3606 is present at a concentration of 5 µM, the NET formation induced by human polyvalent Ig is greater than the NET formation induced by any of the tested concentrations of motavizumab in the absence of human polyvalent Ig. These results thus demonstrate that motavizumab alone is not able to induce NET formation. Motavizumab acts as an enhancer or amplificator of NET formation induced by human polyvalent Ig. Taken together, the data demonstrate that the use of immobilized human polyvalent Ig as described herein can also allow to identify, screen and/or assess agents or drugs which may act as immunostimulators by enhancing neutrophil activation and NET formation.

### Example 3: Evaluation of NET formation by human neutrophils from multiple healthy donors induced in vitro by plate-bound human polyvalent Ig

### Materials and Methods

### Reagents

High-binding, sterile 96-well plates were coated with human polyvalent Ig as described in Example 1, using a coating solution comprising human polyvalent Ig at a concentration ranging from 3 to 300 µg/mL, as indicated.

### Cells and cell culture

Human peripheral blood neutrophils were isolated and cultured as described in Example 1. Blood samples were obtained from 7 healthy human individuals and collected at three independent time points (time points 1, 2, and 3), separated by a least two weeks. Freshly isolated peripheral blood neutrophils were distributed in coated plate as described in Example 1 (7.5×10⁴ cells per well, corresponding to 10⁶ cells/mL of culture medium) and cultured for 16-24 hours in culture medium comprising the fluorescent, cell-impermeant, nucleic acid dye Sytox Green (1 µM).

### Detection

Time-lapse images were acquired by live cell fluorescent microscopy. Quantitative time-course measure of NET formation was performed by computer-assisted image analysis (data are expressed as area under the curve or AUC).

### Results

### Individuals display different susceptibilities to NET formation induced in vitro by human polyvalent Ig

Formation of NETs induced *in vitro* by plate-bound human polyvalent Ig was assessed using human peripheral blood neutrophils isolated from 7 healthy individuals.

Figures **10-16** shows the NET formation induced *in vitro* by plate-bound human polyvalent Ig with neutrophils isolated from each of the 7 healthy individuals, referred to with a number (individual 86 - **Figures 10A-C**, individual 87 - **Figures 11A-C**, individual 88 - **Figures 12A-C**, individual 89 - **Figures 13A-C**, individual 90 - **Figures 14A-C**, individual 85 - **Figures 15A-C**, and individual 91 - **Figures 16A-C**). For each individual, NET formation induced *in vitro* by plate-bound human polyvalent Ig was assessed using neutrophils isolated from three different blood samples obtained at three independent time points (time points 1 - **Figures 10A-16A**, 2 - **Figures 10B-16B**, and 3- **Figures 10C-16C**), separated by a least two weeks. For each individual, NET formation was assessed by contacting neutrophils at a fixed concentration of 7.5×10⁴ cells per well, corresponding to 10⁶ cells/mL of culture medium, with increasing densities of plate-bound human polyvalent Ig (obtained with a coating solution comprising human polyvalent Ig at a concentration of 3, 10, 30, 100 or 300 µg/mL).

Strikingly, as shown on **Figures 10-16**, the extent of NET formation observed in response to the engagement of the Fc receptors at the surface of neutrophils by immobilized human polyvalent Ig varies among individuals. Indeed, depending on the individual, maximal NET formation was obtained with differing densities of plate-bound human polyvalent Ig. The 7 healthy individuals could thus be identified as low, intermediate or high responders depending on the concentration of immobilized human polyvalent Ig required to induce maximal NET formation.

Low responders, such as individuals 86 and 87 (see **Figures 10A-C** and 11A-C, respectively), require a concentration of 100 µg/mL of immobilized human polyvalent Ig (concentration of human polyvalent Ig in the coating solution) to exhibit maximal NET formation. In other words, for low responders, maximal NET formation is observed with a low ratio neutrophils:human polyvalent Ig.

Intermediate responders, such as individuals 88, 89 and 90 (see **Figures 12A-C**, **13A-C** and **14A-C**, respectively), require a concentration of 30 µg/mL of immobilized human polyvalent Ig (concentration of human polyvalent Ig in the coating solution) to exhibit maximal NET formation. In other words, for intermediate responders, maximal NET formation is observed with an intermediate ratio neutrophils:human polyvalent Ig.

High responders, such as individuals 85 and 91 (see **Figures 15A-C** and **16A-C**, respectively), require a concentration of only 10 µg/mL of immobilized human polyvalent Ig (concentration of human polyvalent Ig in the coating solution) to exhibit maximal NET formation. In other words, for high responders, maximal NET formation is observed with a high ratio neutrophils:human polyvalent Ig.

Strikingly, the ratio neutrophils:human polyvalent Ig at which maximal NET formation is observed is consistent for a given individual. Indeed, the responses of each individual are similar over time, and no significant differences are observed with neutrophils isolated from three different blood samples obtained at three independent time points (time points 1, 2, and 3) as illustrated, for example, with Figures **10A-C**, **12A-C**, and **16A-C**.

The results of **Figures 10-16** thus appear to indicate that each individual is characterized by an intrinsic susceptibility to NET formation. Said susceptibility to NET formation can demonstrably be determined with an *in vitro* assay relying on the use of immobilized human polyvalent Ig. Of note, it may be of clinical interest to assess the susceptibility of a subject to NET formation, in particular for a subject suffering from a disease or condition to which neutrophil activation may contribute.

## Claims

1. An *in vitro* method for inducing neutrophil extracellular trap (NET) formation, said method comprising contacting neutrophils in *in vitro* culture with human polyvalent immunoglobulins, wherein said human polyvalent immunoglobulins are immobilized on a solid support.

2. The method according to claim **1**, wherein said method does not comprise contacting the neutrophils in *in vitro* culture with phorbol-12-myristate-13-acetate (PMA).

3. The method according to claim **1** or **2**, wherein the solid support is coated with human polyvalent immunoglobulins using a coating solution comprising human polyvalent immunoglobulins at a concentration of at least about 3 µg/mL.

4. The method according to any one of claims **1** to **3**, wherein the neutrophils are cultured at a concentration of at least about 1x10⁵ neutrophils/mL of culture medium.

5. The method according to any one of claims **1** to **4**, wherein the neutrophils are cultured in a culture medium comprising a cell-impermeant nucleic acid dye.

6. The method according to claim **5**, wherein the method comprises assessing NET formation by image analysis.

7. The method according to claim **5** or **6**, wherein the cell-impermeant nucleic acid dye is fluorescent and wherein the method comprises assessing NET formation by fluorescence detection.

8. An *in vitro* method for screening a drug for its ability to modulate neutrophil extracellular trap (NET) formation, said method comprising:
a) contacting neutrophils in *in vitro* culture with a drug;
b) inducing NET formation by contacting the neutrophils in *in vitro* culture with human polyvalent immunoglobulins immobilized on a solid support according to the method of any one of claims **1** to **7**; and
c) assessing the effect of the drug on NET formation in the *in vitro* culture of neutrophils,
wherein the order of step a) and b) can be inverted.

9. An *in vitro* method for assessing the susceptibility of a subject to neutrophil extracellular trap (NET) formation, said method comprising:
a) contacting an *in vitro* culture of neutrophils previously obtained from a subject with human polyvalent immunoglobulins immobilized on a solid support, thereby inducing NET formation according to the method of any one of claims **1** to **7**; and
b) quantifying NET formation induced in the *in vitro* culture of neutrophils, thereby assessing the susceptibility of the subject to NET formation.

10. The method according to claim **9**, further comprising determining the ratio of neutrophils:human polyvalent immunoglobulins required to induce maximal NET formation in the *in vitro* culture of neutrophils.

11. An *in vitro* method for predicting the response of a subject to a modulator of neutrophil extracellular trap (NET) formation, said method comprising:
a) contacting an *in vitro* culture of neutrophils previously obtained from a subject with a modulator of NET formation;
b) inducing NET formation by contacting the *in vitro* culture of neutrophils with human polyvalent immunoglobulins immobilized on a solid support according to the method of any one of claims **1** to **7**; and
c) assessing the effect of the modulator of NET formation on NET formation in the *in vitro* culture of neutrophils,
wherein the order of step a) and b) can be inverted.

12. An *in vitro* method for monitoring the response of a subject to an immunomodulator, said method comprising:
a) contacting an *in vitro* culture of neutrophils previously obtained from a subject with human polyvalent immunoglobulins immobilized on a solid support, thereby inducing NET formation according to the method of any one of claims **1** to **7**, wherein said subject was treated or is being treated with an immunomodulator;
b) quantifying NET formation induced in the *in vitro* culture of neutrophils; and
c) comparing the NET formation induced in the *in vitro* culture of neutrophils to a reference value, thereby monitoring the response of the subject to the immunomodulator.

13. The method according to claim **12**, wherein the reference value is the NET formation induced in an *in vitro* culture of neutrophils previously obtained from the subject before treatment with the immunomodulator.

14. The method according to any one of claims **9** to **13**, wherein the subject is suffering from a disease or condition to which NET formation may contribute, in particular selected from an inflammatory disease, a thromboembolic diseases, a solid cancer, and a fibrosis.

15. The method according to claim **14**, wherein the disease or condition to which NET formation may contribute is selected from vasculitis, in particular ANCA vasculitis; asthma; rheumatoid arthritis (RA); chronic obstructive pulmonary disease (COPD); systemic lupus erythematous (SLE); systemic sclerosis; adverse cardiovascular events such as myocardial infarction; ischemic stroke; atherosclerosis; venous thrombosis; cancer, in particular solid cancer; fibrosis, for example cystic fibrosis; sepsis; gout; and Alzheimer's disease.
